(19) Europäisches Patentamt
European Patent Office
Office européen des brevets

(11) EP 1 186 287 A1

(12) EUROPEAN PATENT APPLICATION
published in accordance with Art. 158(3) EPC

(43) Date of publication:
13.03.2002 Bulletin 2002/11

(21) Application number: 01917702.1

(22) Date of filing: 30.03.2001

(51) Int Cl.[7]: **A61K 7/06**, A61P 17/14

(86) International application number:
PCT/JP01/02756

(87) International publication number:
WO 01/72268 (04.10.2001 Gazette 2001/40)

(84) Designated Contracting States:
AT BE CH CY DE DK ES FI FR GB GR IE IT LI LU MC NL PT SE TR

(30) Priority: 31.03.2000 JP 2000097542

(71) Applicant: TORAY INDUSTRIES, INC.
Tokyo 103-8666 (JP)

(72) Inventors:
- KUMAGAI, Hiroki
Kamakura-shi, Kanagawa 248-0034 (JP)
- YAMADA, Naohiro
Kamakura-shi, Kanagawa 248-0034 (JP)
- HAYASHI, Ryoji
Fujisawa-shi, Kanagawa 251-0033 (JP)
- MORI, Takeshi
Yokohama-shi, Kanagawa 222-0002 (JP)
- ISOGAYA, Masafumi
Yokohama-shi, Kanagawa 245-0016 (JP)

(74) Representative: Kador & Partner
Corneliusstrasse 15
80469 München (DE)

# (54) HAIR GROWTH OR HAIR FORMATION CONTROLLING AGENTS

(57) An agent for modulating growth or generation of hair, which has an excellent activity for modulating growth or generation of hair, and of which side effect is small, is disclosed. The agent for modulating growth or generation of hair according to the present invention comprises a prostaglandin EP4 receptor ligand as an active ingredient.

EP 1 186 287 A1

## Description

Technical Field

The present invention relates to an agent for modulating growth or generation of hair, which promotes or inhibits growth or generation of hair.

Background Art

Each hair has its own growth cycle (anagen, catagen, telogen and defluxion). However, when generation or growth cycle of each hair is disturbed by a genetic cause or by some other cause, the hair rapidly falls out or conversely grows lengthily and thickly. Abnormal generation of hair includes atrichia and hirsutism, and abnormal hair growth includes alopecia (male pattern alopecia and alopecia areata). These causes are thought to include various factors such as influences by hormones, decrease in blood flow into, hair follicle hypersteatosis, unbalanced diet and stress. Thus, conventional therapy is performed by reducing these causes. For example, therapies of alopecia include inhibition of androgen, increasing in blood flow into hair follicle, pharmacotherapies using compounds which remove unnecessary cuticle and sebum, dietetic treatment and psychotherapies. Since the real cause of alopecia has not been completely clarified, sufficient therapeutic or preventive effect has not been attained. Methods for removing hairs such as body hairs include physical treatments using shavers or depilators, and pharmacotherapies using grainer creams or depilatory creams. However, these methods accompany skin chapping or irritation pain, so that they are not satisfactory as depiliation methods. Thus, development of an agent for modulating growth or generation of hair, which shows sufficient effect, has been demanded for a long time.

It has been disclosed that E type prostaglandins (hereinafter referred to as "PG" for short) and their derivatives promote growth of hair (WO9833497). However, prostaglandin $E_2$ (hereinafter referred to as "$PGE_2$" for short) shows a wide variety of physiological actions such as uterine-contracting action, gastric acid secretion-inhibiting action, gastric mucosa-protecting action, stimulation action of peristalsis of digestive tract, febrifacient action and diarrhea-causing action. Therefore, if $PGE_2$ is used for the purpose of growing or generating hair, these actions act as side effects. physiological actions by $PGE_2$ are expressed by binding of $PGE_2$ to specific receptors. Further, the receptors to which $PGE_2$ bind may be classified into 4 receptor subtypes called EP1, EP2, EP3 and EP4 receptors (Coleman,R.A.et al., Pharmacol. Rev.,46,205-229(1994)). It is also known that each receptor subtype participates in different physiological action. For example, the febrile response by $PGE_2$ is caused by binding of $PGE_2$ to EP3 receptor (Ushikubi F., Nature, 395, 281-284 (1998)). It is known that compounds which specifically bind to EP4 receptor subtype are effective for prevention, therapy or amelioration of immune diseases, asthma, osteodystrophy, apoptosis of neurocytes, hepatopathy, nephritis, hypertension, myocardial ischemia, gastrointestinal disorder, shock and the like (Japanese Laid-open Patent Application (Kokai) No. 10-265454, WO98/55468). However, it is not known that these compounds have hair generation- or hair growth-modulating actions.

Disclosure of the Invention

An object of the present invention is to provide an agent for modulating growth or generation of hair, which has an excellent activity for modulating growth or generation of hair, and of which side effect is small.

The present inventors intensively searched the compounds which have small side effects and which show excellent activities for modulating growth or generation of hair to discover that compounds which strongly act on EP4 receptor and which weakly bind to other subtypes of $PGE_2$ attain this object, thereby completing the present invention.

That is, the present invention provides an agent for modulating growth or generation of hair comprising a prostaglandin EP4 receptor ligand as an active ingredient. The present invention also provides use of a prostaglandin EP4 receptor ligand for production of an agent for modulating growth or generation of hair. The present invention further provides a method for modulating growth or generation of hair comprising administering a prostaglandin EP4 receptor ligand in an amount effective for modulating growth or generation of hair to human or an animal.

The agent for modulating growth or generation of hair according to the present invention has an excellent activity for modulating growth or generation of hair, and its side effects are small.

Best Mode for Carrying Out the Invention

The prostaglandin EP4 receptor ligand is not restricted as long as it acts on prostaglandin EP4 receptor. Examples of such a ligand include 5,6,7-trinor-4,8-inter-m-phenylene $PGI_2$ derivatives of the following Formula (I) and pharmacologically acceptable salts thereof:

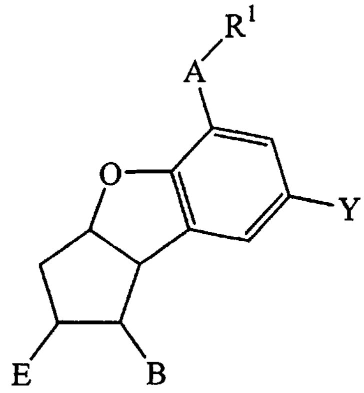

(I)

[wherein
$R^1$ is

(i)

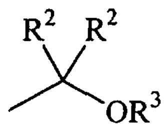

wherein $R^2$ is hydrogen, $C_1$-$C_4$ linear alkyl, $C_3$ or $C_4$ branched alkyl, trifluoromethyl, -C(=O)-$R^4$, or -C(=O)-O-$R^4$, wherein $R^4$ is $C_1$-$C_{12}$ linear alkyl, $C_3$-$C_{14}$ branched alkyl, $C_3$-$C_{12}$ cycloalkyl, $C_7$-$C)_{12}$ aralkyl, phenyl or substituted phenyl (wherein the substituent is at least one fluorine, chlorine, bromine, iodine, trifluoromethyl, $C_1$-$C_4$ alkyl, nitro, cyano, methoxy, phenyl, phenoxy, p-acetamidebenzamide, -CH=N-NH-C(=O)-$NH_2$, -NH-C(=O)-Ph, -NH-C(=O)-$CH_3$ or -NH-C(=O)-$NH_2$), and the two $R^2$s may be the same or different; $R^3$ is hydrogen, $C_1$-$C_4$ alkyl, $C_1$-$C_{12}$ acyl, $C_7$-$C_{16}$ aroyl, $C_7$-$C_{16}$ aralkyl, tetrahydropyranyl, tetrahydrofuranyl, 1-ethoxyethyl, allyl, tert-butyl or tert-butyldimethylsilyl,

(ii) -$COOR^5$

wherein $R^5$ is

(1) hydrogen or pharmacologically acceptable cation,
(2) $C_1$-$C_{12}$ linear alkyl or $C_3$-$C_{14}$ branched alkyl,
(3) -Z-$R^6$
wherein Z is a valence bond, or linear or branched alkylene represented by the formula $C_tH_{2t}$ wherein t represents an integer of 1 to 6, $R^6$ is $C_3$-$C_{12}$ cycloalkyl, or $C_3$-$C_{12}$ cycloalkyl substituted with 1 to 4 $R_7$s wherein $R^7$ is hydrogen or $C_1$-$C_5$ alkyl,
(4) -$(CH_2CH_2O)_nCH_3$
wherein n represents an integer of 1 to 5,
(5) -Z-Ar
wherein Z is defined as the same as the above, Ar is phenyl, α-naphthyl, β-naphthyl, 2-pyridyl, 3-pyridyl, 4-pyridyl, α-furyl, β-furyl, α-thienyl, β-thienyl or substituted phenyl (wherein the substituent is the same as the substituent defined for the substituted phenyl mentioned above),
(6) -$C_tH_{2t}COOR^8$
wherein t is defined as the same as the above, $R^8$ is hydrogen or $C_1$-$C_5$ alkyl,
(7) -$C_tH_{2t}N(R^9)_2$

wherein t is defined as the same as above, $R^9$ is hydrogen or $C_1$-$C_5$ alkyl, and the two $R^9$s may be the same or different,

(8) $-CH(R^{10})-C(=O)-R^{11}$

wherein $R^{10}$ is hydrogen or benzoyl, $R^{11}$ is phenyl, p-bromophenyl, p-chlorophenyl, p-biphenyl, p-nitrophenyl, p-benzamidephenyl or 2-naphthyl,

(9) $-C_pH_{2p}-W-R^{12}$

wherein p represents an integer of 1 to 5, W is -CH=CH-, $-CH=C(R^{13})-$ or -C≡C- wherein $R^{13}$ is $C_1$-$C_{30}$ linear alkyl, $C_3$-$C_{30}$ branched alkyl or $C_7$-$C_{30}$ aralkyl, $R^{12}$ is hydrogen, $C_1$-$C_{30}$ linear alkyl, $C_3$-$C_{30}$ branched alkyl or $C_7$-$C_{30}$ aralkyl, or

(10) $-CH(CH_2OR^{14})_2$

wherein $R^{14}$ is $C_1$-$C_{30}$ alkyl or $C_1$-$C_{30}$ acyl, and the two $R^{14}$s may be the same or different,

(iii)

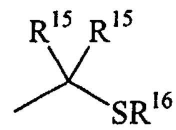

wherein $R^{15}$ is hydrogen, $C_1$-$C_4$ linear alkyl, $C_3$ or $C_4$ branched alkyl, trifluoromethyl, $C(=O)-R^{17}$ or $-C(=O)-O-R^{17}$ wherein $R^{17}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above), and the two $R^{15}$s may be the same or different; $R^{16}$ is hydrogen, $C_1$-$C_{12}$ linear alkyl, $C_3$-$C_{14}$ branched alkyl, phenyl or substituted phenyl (wherein the substituent is the same as the substituent defined for the substituted phenyl mentioned above), or

$-C(=O)-R^{18}$ wherein $R^{18}$ represents $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above),

(iv) $-CH_2-R^{19}$

wherein $R^{19}$ is

(1)

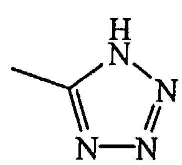

(2)

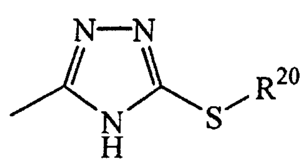

wherein $R^{20}$ is hydrogen, $C_1$-$C_{12}$ linear alkyl, $C_3$-$C_{14}$ branched alkyl, phenyl, substituted phenyl (wherein the substituent is the same as the substituent defined for the substituted phenyl mentioned above), or $-C(=O)-R^{21}$ wherein $R^{21}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl, or substituted phenyl (wherein the substituent is the same as the substituent defined for the substituted phenyl mentioned above),

(3)

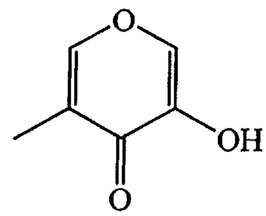

(4)

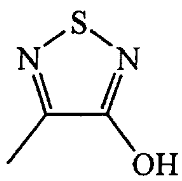

(5)

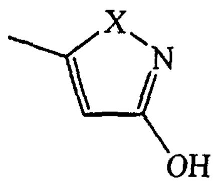

wherein X represents -O- or -S-, or
(6) azide,

(v) $-C(R^{22})_3$

wherein $R^{22}$ represents hydrogen, fluorine, chlorine, bromine, iodine, cyano or $C_1$-$C_4$ alkyl, and all of the $R^{22}$s may be the same or different,

(vi)

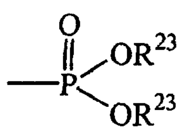

wherein $R^{23}$ represents hydrogen, $C_1$-$C_4$ alkyl, phenyl, substituted phenyl (wherein the substituent is the same as the substituent defined for the substituted phenyl mentioned above), $-CH_2-OR^{24}$ (wherein $R^{24}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl, or substituted phenyl (wherein the substituent is the same as the substituent defined for the substituted phenyl mentioned above), or pharmacologically acceptable cation, and the two $R^{23}$s may be the same or different,

(vii) $-N(R^{25})_2$

wherein $R^{25}$ is hydrogen, $C_1$-$C_{12}$ linear alkyl, $C_3$-$C_{14}$ branched alkyl, $C_3$-$C_{12}$ cycloalkyl, $C_4$-$C_{13}$ cycloalkylalkyl, $C_7$-$C_{12}$ aralkyl, $-C(=O)-R^{26}$,$-C(=O)-O-R^{26}$, $-SO_2-R^{26}$, phenyl or substituted phenyl (wherein the substituent is the same as the substituent defined for the substituted phenyl mentioned above), $R^{26}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same

as the substituent defined for the substituted phenyl mentioned above), the two R $^{25}$s may be the same or different (when one of the $R^{25}$s is
$-SO_2-R^{26}$, the other $R^{25}$ is not $-SO_2-R^{26}$),

(viii) $-(C(=O)CH_2)_k-H$

wherein k represents an integer of 1 or 2, or

(ix) $-C(=O)-N(R^{27})_2$

wherein $R^{27}$ is hydrogen, $C_1$-$C_{12}$ alkyl, C3-$C_{12}$ cycloalkyl, phenyl, substituted phenyl (wherein the substituent is the same as the substituent defined for the substituted phenyl mentioned above), $C_4$-$C_{13}$ cycloalkylalkyl, $C_7$-$C_{12}$ aralkyl, cyano or $-SO_2-R^{28}$ wherein $R^{28}$ is $C_1$-$C_{12}$ alkyl, $C_3$-$C_{12}$ cycloalkyl, phenyl, substituted phenyl (wherein the substituent is the same as the substituent defined for the substituted phenyl mentioned above), $C_4$-$C_{13}$ cycloalkylalkyl, or $C_7$-$C_{12}$ aralkyl, and the two $R^{27}$s may be the same or different (when one of the $R^{27}$s is $-SO_2-R^{28}$, the other $R^{27}$ is not $-SO_2-R^{28}$);
Y is hydrogen, $C_1$-$C_4$ alkyl, fluorine, chlorine, bromine, formyl, methoxy or nitro;
B is

(i)

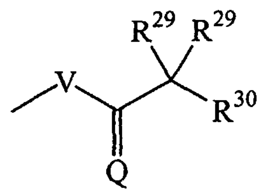

wherein V is

(1) $-CH_2CH_2-$,

(2) $-C{\equiv}C-$,

or

(3) $-CH=C(R^{31})-$

wherein $R^{31}$ is hydrogen, $C_1$-$C_5$ alkyl, fluorine, chlorine, bromine or iodine,
Q is

(1) =O

(2)

$—R^{32}$

$—OR^{33}$

or

(3)

—$R^{32}$

—$R^{32}$

wherein $R^{32}$ is hydrogen, $C_1$-$C_4$ linear alkyl, $C_3$ or $C_4$ branched alkyl, trifluoromethyl, -C(=O)-$R^{34}$, or -C(=O)-O-$R^{34}$ wherein $R^{34}$ represents $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above); $R^{33}$ is hydrogen, $C_1$-$C_4$ alkyl, $C_1$-$C_{12}$ acyl, $C_7$-$C_{16}$ aroyl, $C_7$-$C_{16}$ aralkyl, tetrahydropyranyl, tetrahydrofuranyl, 1-ethoxyethyl, allyl, tert-butyl or tert-butyldimethylsilyl, and the two $R^{32}$s may be the same or different; $R^{29}$ is hydrogen, fluorine, chlorine, bromine, iodine, cyano or $C_1$-$C_4$ alkyl, and the two $R^{29}$s may be the same or different;
$R^{30}$ is

(1) -Z-$R^{35}$

wherein Z is defined as the same as the above, $R^{35}$ is $C_1$-$C_{12}$ linear alkyl, $C_3$-$C_{14}$ branched alkyl, $C_3$-$C_{12}$ cycloalkyl, $C_4$-$C_{13}$ cycloalkylalkyl, $C_3$-$C_{12}$ cycloalkyl substituted with 1 to 4 $R^{36}$s (wherein $R^{36}$ is hydrogen or $C_1$-$C_5$ alkyl), $C_4$-$C_{13}$ cycloalkylalkyl substituted with 1 to 3 $R^{36}$s (wherein $R^{36}$ is defined as the same as the above), phenyl, substituted phenyl (wherein the substituent is the same as the substituent defined for the substituted phenyl mentioned above), α-naphthyl, β-naphthyl, 2-pyridyl, 3-pyridyl, 4-pyridyl, α-furyl, (β-furyl, α-thienyl or β-thienyl,

(2) -Z-O-$R^{35}$

wherein Z and $R^{35}$ are defined as the same as the above,

(3) -Z-CH=C($R^{35}$)$_2$

wherein Z and $R^{35}$ are defined as the same as the above, and the two $R^{35}$s may be the same or different, or

(4) -Z-C≡C-$R^{35}$

wherein Z and $R^{35}$ are defined as the same as the above,

(ii)

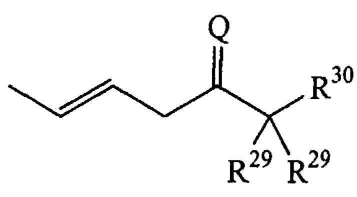

wherein Q, $R^{29}$ and $R^{30}$ are defined as the same as the above, and the two $R^{29}$s may be the same or different, or

(iii)

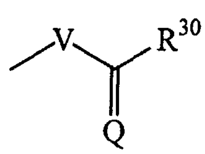

wherein V, Q and $R^{30}$ are defined as the same as the above;
E represents hydrogen or $-OR^{33}$ wherein $R^{33}$ is defined as the same as the above;
A is

(i)

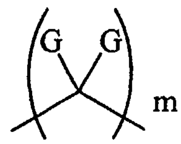

wherein m represents an integer of 0 to 5, G represents hydrogen, fluorine, chlorine, bromine, iodine, trifuluoromethyl, $C_1$-$C_4$ linear alkyl or $C_3$-$C_6$ branched alkyl, and all Gs may be the same or different,

(ii)

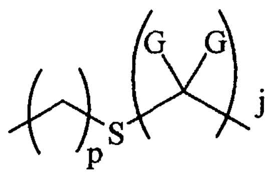

wherein j represents an integer of 1 to 4, p represents an integer of 0 or 1, G is defined as the same as the above, and all Gs may be the same or different,

(iii) $-CH=CH-CH_2-$,

(iv) $-CH_2-CH=CH-$,

(v) $-CH_2-O-CH_2-$,

(vi) $-O-CH_2-$,

(vii) $-C\equiv C-$,

or

(viii) -C=C- (trans)].

Examples about the above-described Formula (I) will now be described. However, the present invention is not restricted thereto.

Examples of $R^2$ include hydrogen, methyl, ethyl, propyl, butyl, isopropyl, isobutyl, tert-butyl, trifluoromethyl, acetyl, propionyl, benzoyl, phenylacetyl, methoxycarbonyl and ethoxycarbonyl. Among these, preferred are hydrogen, methyl, ethyl, propyl, butyl, isopropyl, isobutyl, tert-butyl and trifluoromethyl, more preferred are hydrogen, methyl, ethyl, propyl, butyl and trifluoromethyl, and still more preferred are hydrogen and methyl.

Examples of $R^3$ include hydrogen, methyl, ethyl, propyl, butyl, isopropyl, isobutyl, tert-butyl, formyl, acetyl, propionyl, butyloyl, benzoyl, phenylacetyl, 3-phenylpropionyl, 10-phenyldecanoyl, p-phenylbenzoyl, α-naphthoyl, β-naphthoyl, benzyl, phenetyl, 3-phenylpropyl, 6-phenylhexyl, 10-phenyldecyl, p-methylbenzyl, p-ethylbenzyl, p-propylbenzyl, p-pentylbenzyl, p-nonylbenzyl, 3,5-dimethylbenzyl, 3,5-diethylbenzyl, 3,5-dibutylbenzyl, p-phenylbenzyl, tetrahydropyranyl, tetrahydrofuranyl, 1-ethoxyethyl, allyl, tert-butyl and tert-butyldimethylsilyl. Among these, preferred are

hydrogen, acetyl, propionyl, butyloyl, benzoyl, phenylacetyl, 3-phenylpropionyl, 10-phenyldecanoyl, p-phenylbenzoyl, α-naphthoyl, β-naphthoyl, tetrahydropyranyl, tetrahydrofuranyl, 1-ethoxyethyl, allyl and tert-butyldimethylsilyl, more preferred are hydrogen, acetyl, tetrahydropyranyl, tetrahydrofuranyl and tert-butyldimethylsilyl, and still more preferred is hydrogen.

Examples of $R^4$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Preferred examples of $R^5$ include hydrogen, lithium ion, sodium ion, potassium ion, magnesium ion, calcium ion, cations derived from amines (e.g., methylamine, dimethylamine, triethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, 1-methylpiperidine, 4-ethylmorpholine, 1-isopropylpyrrolidine, 2-methylpyrrolidine, 1,4-dimethylpiperazine, 2-methylpiperidine, mono-, di-, and triethanolamine, ethyldiethanolamine, N-butylethanolamine, 2-amino-1-butanol, 2-amino-2-ethyl-1,3-propanediol, tris(hydroxymethyl)aminomethane, N-phenylethanolamine, N-(p-tert-aminophenyl)diethanolamine, galactamine, N-methylglutamine, N-methylglucosamine, ephedrine, phenylephrine, epinephrine, procaine, lysine and arginine), methyl, ethyl, propyl, butyl, pentyl, hexyl, heptyl, octyl and dodecyl. Among these, more preferred are hydrogen, lithium ion, sodium ion, potassium ion, magnesium ion, calcium ion, cations derived from amines (triethylamine, ethylenediamine, diethylenetriamine, and mono-, di- and triethanolamine), methyl, ethyl, propyl and butyl, and still more preferred are hydrogen, sodium ion and methyl.

Examples of $R^6$ include cyclopropyl, cyclobutyl, cyclopentyl, cyclohexyl, cycloheptyl, cyclooctyl, cyclononyl, cyclododecyl, 2-methylcyclopropyl, 3-methylcyclobutyl, 3-methylcyclopentyl, 4-methylcyclohexyl, 4-methylcycloheptyl, 5-methylcyclooctyl and 5-methylcyclononyl. Among these, preferred are cyclopentyl, cyclohexyl, cycloheptyl, 3-methylcyclopentyl, 4-methylcyclohexyl and 4-methylcycloheptyl, and more preferred are cyclohexyl and 4-methylcyclohexyl, still more preferred is cyclohexyl.

Examples of $R^7$ include hydrogen, methyl, ethyl, propyl, butyl, isopropyl, isobutyl and tert-butyl. Among these, preferred are hydrogen, methyl, ethyl, isopropyl and tert-butyl, still more preferred are hydrogen, methyl and ethyl, and still more preferred are hydrogen and methyl.

Examples of $R^8$ include hydrogen, methyl, ethyl, propyl, butyl, isopropyl, isobutyl and tert-butyl. Among these, preferred are hydrogen, methyl, ethyl, isopropyl and tert-butyl, still more preferred are hydrogen, methyl and ethyl, and still more preferred are hydrogen and methyl.

Examples of $R^9$ include hydrogen, methyl, ethyl, propyl, butyl, isopropyl, isobutyl and tert-butyl. Among these, preferred are hydrogen, methyl, ethyl, isopropyl and tert-butyl, still more preferred are hydrogen, methyl and ethyl, and still more preferred are hydrogen and methyl.

$R^{10}$ represents hydrogen or benzoyl, and hydrogen is especially preferred.

$R^{11}$ represents phenyl, p-bromophenyl, p-chlorophenyl, p-biphenyl, p-nitrophenyl, p-benzamidephenyl or 2-naphthyl, preferably phenyl, p-bromophenyl, p-chlorophenyl, p-biphenyl or p-nitrophenyl, more preferably phenyl, p-chlorophenyl or p-biphenyl, still more preferably phenyl.

Examples of $R^{12}$ include hydrogen, methyl, ethyl, propyl, butyl, pentyl, hexyl, heptyl, octyl, dodecyl, isopropyl, isobutyl, tert-butyl, 4-methylpentyl, 5-methylhexyl, 6-methylheptyl, 7-methyloctyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl and 3,5-diethylbenzyl. Among these, preferred are hydrogen, methyl, ethyl, propyl, isopropyl, isobutyl, tert-butyl, benzyl, phenetyl and 3-phenylpropyl, more preferred are hydrogen, methyl, ethyl, isopropyl, isobutyl, benzyl and phenetyl, and still more preferred are hydrogen, methyl, isopropyl and benzyl.

Examples of R13 include hydrogen, methyl, ethyl, propyl, butyl, pentyl, hexyl, heptyl, octyl, dodecyl, isopropyl, isobutyl, tert-butyl, 4-methylpentyl, 5-methylhexyl, 6-methylheptyl, 7-methyloctyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl and 3,5-diethylbenzyl. Among these, preferred are hydrogen, methyl, ethyl, propyl, isopropyl, isobutyl, tert-butyl, benzyl, phenetyl and 3-phenylpropyl, more preferred are hydrogen, methyl, ethyl, isopropyl, isobutyl, benzyl and phenetyl, and still more preferred are hydrogen, methyl, isopropyl and benzyl.

Examples of $R^{14}$ include methyl, ethyl, propyl, butyl, pentyl, hexyl, octyl, hexadecanyl, octaeicosanyl, acetyl, octanoyl, decanoyl, palmitoyl, eicosanoyl and hexaeicosanoyl. Among these, preferred are methyl, ethyl, propyl, acetyl, octanoyl and decanoyl, more preferred are methyl, ethyl, acetyl and octanoyl, and still more preferred are methyl and acetyl.

Examples of $R^{15}$ include hydrogen, methyl, ethyl, propyl, butyl, isopropyl, isobutyl, tert-butyl, trifluoromethyl, acetyl, propionyl, benzoyl, phenylacetyl, methoxycarbonyl and ethoxycarbonyl. Among these, preferred are hydrogen, methyl, ethyl, propyl, butyl, isopropyl, isobutyl, tert-butyl and trifluoromethyl, more preferred are hydrogen, methyl, ethyl, propyl, butyl and trifluoromethyl, and still more preferred are hydrogen and methyl.

Examples of $R^{16}$ include hydrogen, methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, phenyl, p-fluorophenyl, p-nitrophenyl, p-methoxyphenyl, acetyl and propionyl. Among these, preferred are hydrogen, methyl, ethyl, propyl, butyl, phenyl, acetyl and propionyl, more preferred are hydrogen, methyl, ethyl, propyl, phenyl and acetyl, and still more preferred are hydrogen, methyl, phenyl and acetyl.

Examples of $R^{17}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{18}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{20}$ include hydrogen, methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, phenyl, p-fluorophenyl, p-nitrophenyl, p-methoxyphenyl, acetyl and propionyl. Among these, preferred are hydrogen, methyl, ethyl, propyl, butyl, phenyl, acetyl and propionyl, more preferred are hydrogen, methyl, ethyl, propyl, phenyl and acetyl, and still more preferred are hydrogen, methyl, phenyl and acetyl.

Examples of $R^{21}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{22}$ include hydrogen, fluorine, chlorine, bromine, iodine, cyano, methyl, ethyl, propyl, butyl, isopropyl, isobutyl and tert-butyl. Among these, preferred are hydrogen, fluorine, cyano, methyl, ethyl, propyl, butyl, isopropyl, isobutyl and tert-butyl, more preferred are hydrogen, fluorine, methyl, ethyl and isopropyl, and still more preferred are hydrogen, fluorine and methyl.

Examples of $R^{23}$ include hydrogen, methyl, ethyl, propyl, butyl, isopropyl, isobutyl, tert-butyl, phenyl, p-fluorophenyl, p-nitrophenyl, p-methoxyphenyl, methoxymethyl, ethoxymethyl, lithium ion, sodium ion, potassium ion, magnesium ion, calcium ion, and cations derived from amines (e.g., methylamine, dimethylamine, triethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, 1-methylpiperidine, 4-ethylmorpholine, 1-isopropylpyrrolidine, 2-methylpyrrolidine, 1,4-dimethylpiperazine, 2-methylpiperidine, mono-, di-, and triethanolamine, ethyldiethanolamine, N-butylethanolamine, 2-amino-1-butanol, 2-amino-2-ethyl-1,3-propanediol, tris(hydroxymethyl)aminomethane, N-phenylethanolamine, N-(p-tert-amylphenyl)diethanolamine, galactamine, N-methylglutamine, N-methylglucosamine, ephedrine, phenylephrine, epinephrine, procaine, lysine and arginine). Among these, preferred are hydrogen, methyl, ethyl, propyl, butyl, isopropyl, isobutyl, tert-butyl, lithium ion, sodium ion, potassium ion, magnesium ion, calcium ion, and cations derived from amines (triethylamine, ethylenediamine, diethylenetriamine, and mono-, di-, and triethanolamine), more preferred are hydrogen, methyl, isopropyl, lithium ion, sodium ion, potassium ion, magnesium ion and calcium ion, and still more preferred are hydrogen, methyl and sodium ion.

Examples of $R^{24}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred

are methyl, ethyl and phenyl, and still more preferred are methyl and phenyl.

Examples of $R^{25}$ include hydrogen, methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, cyclopropylmethyl, cyclobutylmethyl, cyclopentylmethyl, cyclohexylmethyl, cycloheptylmethyl, cyclododecylmethyl, 2-cyclopropylethyl, 2-cyclobutylethyl, 2-cyclopentylethyl, 2-cyclohexylethyl, 2-cycloheptylethyl, 3-cyclopropylpropyl, 3-cyclobutylpropyl, 3-cyclopentylpropyl, 3-cyclopentylpropyl, 3-cyclohexylpropyl, 3-cycloheptylpropyl, 6-cyclopropylhexyl, 6-cyclobutylhexyl, 6-cyclopentylhexyl, 6-cyclohexylhexyl, 6-cycloheptylhexyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, acetyl, propionyl, benzoyl, phenylacetyl, methoxycarbonyl, ethoxycarbonyl, methylsulfonyl, ethylsulfonyl, phenylsulfonyl, phenyl, p-fluorophenyl, p-nitrophenyl and p-methoxyphenyl. Among these, preferred are hydrogen, p-methylbenzyl, p-ethylbenzyl, p-propylbenzyl, p-pentylbenzyl, 3,5-dimethylbenzyl, 3,5-diethylbenzyl, acetyl, propionyl, benzoyl, pyhenylacetyl, methoxycarbonyl, ethoxycarbonyl, methylsulfonyl, ethylsulfonyl, phenylsulfonyl, phenyl, p-fluorophenyl, p-nitrophenyl and p-methoxyphenyl, more preferred are hydrogen, acetyl, propionyl, benzoyl, phenylacetyl, methylsulfonyl, ethylsulfonyl and phenylsulfonyl, and still more preferred are hydrogen, acetyl, benzoyl, methylsulfonyl and phenylsulfonyl.

Examples of $R^{26}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{27}$ include hydrogen, methyl, ethyl, propyl, butyl, pentyl, hexyl, heptyl, octyl, dodecyl, cyclopropyl, cyclobutyl, cyclopentyl, cyclohexyl, cycloheptyl, cyclooctyl, cyclononyl, cyclododecyl, cyclopropylmethyl, cyclobutylmethyl, cyclopentylmethyl, cyclohexylmethyl, cycloheptylmethyl, cyclododecylmethyl, 2-cyclopropylethyl, 2-cyclobutylethyl, 2-cyclopentylethyl, 2-cyclohexylethyl, 2-cycloheptylethyl, 3-cyclopropylpropyl, 3-cyclobutylpropyl, 3-cyclopentylpropyl, 3-cyclopentylpropyl, 3-cyclohexylpropyl, 3-cycloheptylpropyl, 6-cyclopropylhexyl, 6-cyclobutylhexyl, 6-cyclopentylhexyl, 6-cyclohexylhexyl, 6-cycloheptylhexyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, cyano, methylsulfonyl, ethylsulfonyl, phenylsulfonyl, phenyl, p-fluorophenyl, p-nitrophenyl and p-methoxyphenyl. Among these, preferred are hydrogen, p-methylbenzyl, p-ethylbenzyl, p-propylbenzyl, p-pentylbenzyl, 3,5-dimethylbenzyl, 3,5-diethylbenzyl, cyano, methylsulfonyl, ethylsulfonyl, phenylsulfonyl, phenyl, p-fluorophenyl, p-nitrophenyl and p-methoxyphenyl, more preferred are hydrogen, cyano, methylsulfonyl, ethylsulfonyl and phenylsulfonyl, and still more preferred are hydrogen, cyano, methylsulfonyl and phenylsulfonyl.

Examples of $R^{28}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl,

1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{29}$ include hydrogen, fluorine, chlorine, bromine, iodine, cyano, methyl, ethyl, propyl, butyl, isopropyl, isobutyl and tert-butyl. Among these, preferred are hydrogen, fluorine, cyano, methyl, ethyl, propyl, butyl, isopropyl, isobutyl and tert-butyl, more preferred are hydrogen, fluorine, methyl, ethyl and isopropyl, and still more preferred are hydrogen, fluorine and methyl.

Examples of $R^{31}$ include hydrogen, methyl, ethyl, propyl, butyl, isopropyl, isobutyl, tert-butyl, fluorine, chlorine, bromine and iodine. Among these, preferred are hydrogen, methyl, ethyl, isopropyl, tert-butyl and fluorine, more preferred are hydrogen, methyl and ethyl, and still more preferred are hydrogen and methyl.

Examples of $R^{32}$ include hydrogen, methyl, ethyl, propyl, butyl, isopropyl, isobutyl, tert-butyl, trifluoromethyl, acetyl, propionyl, benzoyl, phenylacetyl, methoxycarbonyl and ethoxycarbonyl. Among these, preferred are hydrogen, methyl, ethyl, propyl, butyl, isopropyl, isobutyl, tert-butyl and trifluoromethyl, more preferred are hydrogen, methyl, ethyl, propyl, butyl and trifluoromethyl, and still more preferred are hydrogen and methyl.

Examples of $R^{33}$ include hydrogen, methyl, ethyl, propyl, butyl, isopropyl, isobutyl, tert-butyl, formyl, acetyl, propionyl, butyloyl, benzoyl, phenylacetyl, 3-phenylpropionyl, 10-phenyldecanoyl, p-phenylbenzoyl, α-naphthoyl, β-naphthoyl, benzyl, phenetyl, 3-phenylpropyl, 6-phenylhexyl, 10-phenyldecyl, p-methylbenzyl, p-ethylbenzyl, p-propylbenzyl, p-pentylbenzyl, p-nonylbenzyl, 3,5-dimethylbenzyl, 3,5-diethylbenzyl, 3,5-dibutylbenzyl, p-phenylbenzyl, tetrahydropyranyl, tetrahydrofuranyl, 1-ethoxyethyl, allyl, tert-butyl and tert-butyldimethylsilyl. Among these, preferred are hydrogen, acetyl, propionyl, butyloyl, benzoyl, phenylacetyl, 3-phenylpropionyl, 10-phenyldecanoyl, p-phenylbenzoyl, α-naphthoyl, β-naphthoyl, tetrahydropyranyl, tetrahydrofuranyl, 1-ethoxyethyl, allyl and tert-butyldimethylsilyl, more preferred are hydrogen, acetyl, tetrahydropyranyl, tetrahydrofuranyl and tert-butyldimethylsilyl, and still more preferred is hydrogen.

Examples of $R^{34}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, benzyl, phenetyl, 3-phenylpropyl, 6-phenylhexyl, p-methylbenzyl, p-ethylbenzyl, p-propylbenzyl, p-pentylbenzyl, 3,5-dimethylbenzyl, 3,5-diethylbenzyl, phenyl, p-fluorophenyl, p-nitrophenyl and p-methoxyphenyl. Among these, preferred are methyl, ethyl, propyl, butyl and phenyl, more preferred are methyl, ethyl and phenyl, and still more preferred are methyl and phenyl.

Examples of $R^{35}$ include methyl, ethyl, propyl, butyl, pentyl, hexyl, heptyl, octyl, dodecyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1-methyloctyl, 2-methyloctyl, 3-methyloctyl, 4-methyloctyl, 5-methyloctyl, 6-methyloctyl, 7-methyloctyl, 1-methylnonyl, 2-methylnonyl, 1-methyldecanyl, 2-methyldecanyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, 1,1-dimethylheptyl, 2,2-dimethylheptyl, 3,3-dimethylheptyl, 4,4-dimethylheptyl, 5,5-dimethylheptyl, 6,6-dimethylheptyl, 1,1-dimethyloctyl, 2,2-dimethyloctyl, 3,3-dimethyloctyl, 1,1-dimethylnonyl, 2,2-dimethylnonyl, 3,3-dimethylnonyl, 1,1-dimethyldecanyl, 2,2-dimethyldecanyl, 3,3-dimethyldecanyl, 1,1,2,2-tetramethylpentyl, 1,1,3,3-tetramethylpentyl, 1,1,2,2-tetramethylhexyl, 1,1,3,3-tetramethylhexyl, 2,2,3,3-tetramethylhexyl, cyclopropyl, cyclobutyl, cyclopentyl, cyclohexyl, cycloheptyl, cyclooctyl, cyclononyl, cyclododecyl, cyclopropylmethyl, cyclobutylmethyl, cyclopentylmethyl, cyclohexylmethyl, cycloheptylmethyl, cyclododecylmethyl, 2-cyclopropylethyl, 2-cyclobutylethyl, 2-cyclopentylethyl, 2-cyclohexylethyl, 2-cycloheptylethyl, 3-cyclopropylpropyl, 3-cyclobutylpropyl, 3-cyclopentylpropyl, 3-cyclopentylpropyl, 3-cyclohexylpropyl, 3-cycloheptylpropyl, 6-cyclopropylhexyl, 6-cyclobutylhexyl, 6-cyclopentylhexyl, 6-cyclohexylhexyl, 6-cycloheptylhexyl, 2-methylcyclopropyl, 3-methylcyclobutyl, 3-methylcyclopentyl, 4-methylcyclohexyl, 4-methylcycloheptyl, 5-methylcyclooctyl, 5-methylcyclononyl, 2-methylcyclopropylmethyl, 3-methylcyclobutylmethyl, 3-methylcyclopentylmethyl, 4-methylcyclohexylmethyl, 4-methylcycloheptylmethyl, 5-methylcyclooctylmethyl, 5-methylcyclononylmethyl, phenyl, p-fluorophenyl, p-nitrophenyl, p-methoxyphenyl, α-naphthyl, β-naphthyl, 2-pyridyl, 3-pyridyl, 4-pyridyl, α-furyl, (β-furyl, α-thienyl and β-thienyl. Among these, preferred are methyl, ethyl, propyl, butyl, pentyl, hexyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 1-methylheptyl, 2-methylheptyl, 3-methylheptyl, 4-methylheptyl, 5-methylheptyl, 6-methylheptyl, 1,1-dimethylbutyl, 2,2-dimethylbutyl, 3,3-dimethylbutyl, 1,1-dimethylpentyl, 2,2-dimethylpentyl, 3,3-dimethylpentyl, 4,4-dimethylpentyl, 1,1-dimethylhexyl, 2,2-dimethylhexyl, 3,3-dimethylhexyl, 4,4-dimethylhexyl, 5,5-dimethylhexyl, cyclopropyl, cyclobutyl, cyclopentyl, cyclohexyl, cycloheptyl, cyclooctyl, cyclononyl, cyclododecyl, cyclopropylmethyl, cyclobutylmethyl, cyclopentylmethyl, cyclohexylmethyl, 2-methylcyclopropyl, 3-methylcyclobutyl, 3-methylcyclopentyl, 4-methylcyclohexyl, 4-methylcycloheptyl, 5-methylcyclooctyl, 5-methylcyclononyl, 2-methylcyclopropylmethyl, 3-methylcyclobutylmethyl, 3-methylcyclopentylmethyl, phenyl, p-fluorophenyl, p-nitrophenyl and p-methoxyphenyl, more preferred are methyl, ethyl, propyl, butyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1,1-dimethylbutyl, 2,2-dimethylbutyl, 3,3-dimethylbutyl, cyclopropyl, cyclobutyl, cyclopentyl, cyclohexyl, cycloheptyl, cyclooctyl, cyclononyl, cyclopropylmethyl, cyclobutylmethyl, cyclopentylmethyl, cyclohexylmethyl, 2-methylcyclopropyl, 3-methylcyclobutyl, 3-methylcyclopentyl, 4-methylcyclohexyl, 4-methylcycloheptyl, 5-methylcyclooctyl, 2-methylcyclopropylmethyl, 3-methylcyclobutylmethyl, 3-methylcyclopentylmethyl and phenyl, and still more preferred are cyclopentyl, cyclohexyl, cycloheptyl and phenyl.

Examples of $R^{36}$ include hydrogen, methyl, ethyl, propyl, butyl, isopropyl, isobutyl and tert-butyl. Among these, preferred are hydrogen, methyl, ethyl, isopropyl and tert-butyl, more preferred are hydrogen, methyl and ethyl, and still more preferred are hydrogen and methyl.

Examples of G include hydrogen, fluorine, chlorine, bromine, iodine, trifluoromethyl, methyl, ethyl, propyl, butyl, isopropyl, isobutyl, tert-butyl, 1-methylbutyl, 2-methylbutyl, 3-methylbutyl, 1-methylpentyl, 2-methylpentyl, 3-methylpentyl, 4-methylpentyl, 1,1-dimethylbutyl, 2,2-dimethylbutyl and 3,3-dimethylbutyl. Among these, preferred are hydrogen, fluorine, chlorine, bromine, iodine, trifluoromethyl, methyl, ethyl, propyl and butyl, more preferred are hydrogen, fluorine, methyl, ethyl, propyl and butyl, and still more preferred are hydrogen and fluorine.

Examples of Y include hydrogen, methyl, ethyl, propyl, butyl, isopropyl, isobutyl, tert-butyl, fluorine, chlorine, bromine, formyl, methoxy and nitro. Among these, preferred are hydrogen, methyl, fluorine, chlorine, bromine, and formyl, more preferred are hydrogen, methyl and fluorine, and still more preferred is hydrogen.

Among the 5,6,7-trinor-4,8-inter-m-phenylene PGI2 derivatives represented by Formula (I), preferred are those wherein $R^1$ , Y, E and A represent the same meanings as described above, B is

(i)

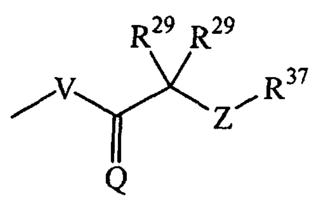

wherein V, Q, $R^{29}$ and Z is the following in the definition of claim 2, the two $R^{29}$s may be the same or different, $R^{37}$ is $C_3$-$C_{12}$ cycloalkyl, $C_4$-$C_{13}$ cycloalkylalkyl, $C_3$-$C_{12}$ cycloalkyl substituted with 1 to 4 $R^{38}$s (wherein $R^{38}$ is hydrogen or $C_1$-$C_5$ alkyl), $C_4$-$C_{13}$ cycloalkylalkyl substituted with 1 to 3 $R^{38}$s (wherein $R^{38}$ is defined as the same as the above), phenyl, substituted phenyl (wherein the substituent is the same as the substituent defined for the substituted phenyl in claim 2), α-naphthyl, β-naphthyl, 2-pyridyl, 3-pyridyl, 4-pyridyl, α-furyl, β-filryl, α-thienyl or β-thienyl,

(ii)

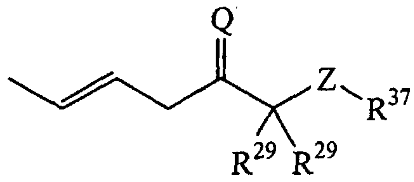

wherein Q, $R^{29}$, Z and $R^{37}$ are defined as the same as the above, and the two $R^{29}$s may be the same or different, or

(iii)

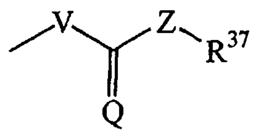

wherein V, Q, Z and $R^{37}$ are defined as the same as the above.

Among these, especially preferred are those represented by the following Formula (II):

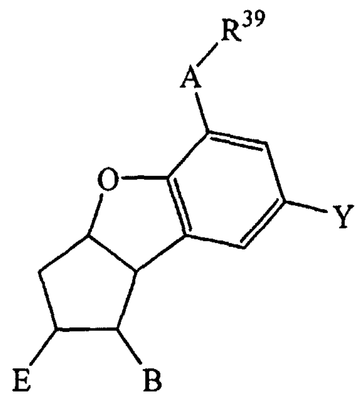

(II)

[wherein $R^{39}$ is

(i)

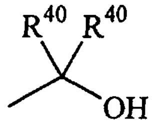

wherein $R^{40}$ is hydrogen, $C_1$-$C_4$ linear alkyl or trifluoromethyl, the two $R^{40}$ may be the same or different,

(ii) $-COOR^{41}$

wherein $R^{41}$ is hydrogen, a pharmacologically acceptable cation or $C_1$-$C_{12}$ linear alkyl,

(iii)

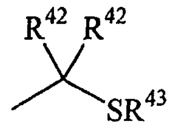

wherein $R^{42}$ is hydrogen, $C_1$-$C_4$ linear alkyl or trifluoromethyl, the two $R^{42}$s may be the same or different, $R^{43}$ is hydrogen, $C_1$-$C_4$ linear alkyl, phenyl, or $-C(=O)-R^{44}$
wherein $R^{44}$ represents $C_1$-$C_4$ linear alkyl,

(iv) $-CH_2-R^{45}$

wherein $R^{45}$ is

(1)

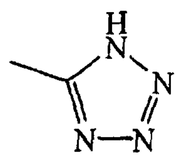

(2)

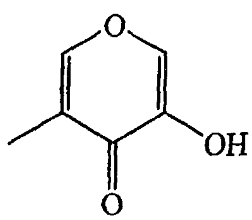

or

(3)

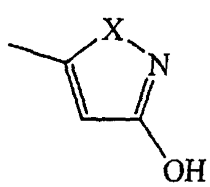

wherein X is the following in the definition of claim 2,

(v) $-C(R^{46})_3$

wherein $R^{46}$ is hydrogen, fluorine, cyano or $C_1$-$C_4$ alkyl, and all $R^{46}$s may be the same or different,

(vi)

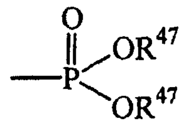

wherein $R^{47}$ is hydrogen, $C_1$-$C_4$ alkyl, or a pharmacologically acceptable cation, and the two $R^{47}$s may be the same or different, or

(vii) $-N(R^{48})_2$

wherein $R^{48}$ is hydrogen, $-C(=O)-R^{49}$ or $-SO_2-R^{49}$ wherein $R^{49}$ is $C_1$-$C_4$ linear alkyl or phenyl, and the two $R^{48}$s may be the same or different (when one of $R^{48}$s is $-SO_2-R^{49}$, the other $R^{48}$ is not $-SO_2-R^{49}$),
Y is hydrogen, fluorine, chlorine or bromine,
B is

(i)

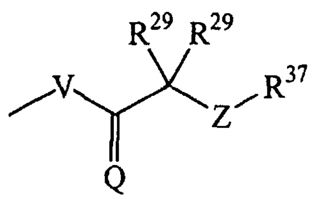

wherein V is

(1) $-CH_2CH_2-$,

(2) $-C{\equiv}C-$,

or

(3) $-CH=CH-$,

Q is

(1) $=O$,

(2)

$—R^{50}$

$—OR^{33}$

or

(3)

—R$^{50}$

—R$^{50}$

wherein $R^{50}$ is hydrogen, $C_1$-$C_4$ linear alkyl, $C_3$ or $C_4$ branched alkyl, or trifluoromethyl, $R^{33}$ represents the following in the definition of claim 2, the two $R^{50}$s may be the same or different, $R^{29}$ represents the following in the definition of claim 2, and the two $R^{29}$s may be the same or different, Z represents the following in the definition of claim 2, and $R^{37}$ represents the following in the definition of claim 3,

(ii)

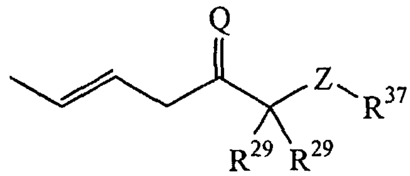

wherein Q, $R^{29}$, Z and $R^{37}$ are defined as the same as the above, and the two $R^{29}$s may be the same or different, or

(iii)

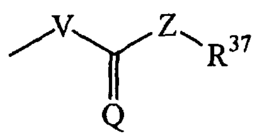

wherein V, Q, Z and $R^{37}$ are defined as the same as the above,
E represents the following in the definition of claim 2,
A is

(i)

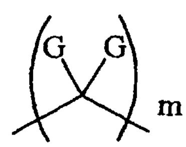

wherein m is an integer of 0 to 3, G is hydrogen, fluorine, chlorine, bromine, iodine, trifluoromethyl or $C_1$-$C_4$ linear alkyl, and all Gs may be the same or different,

(ii)

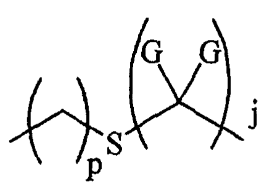

wherein j is an integer of 1 or 2, p represents the following in the definition of claim 2, G is defined as the same as the above, and all Gs may be the same or different,

(iii) -CH=CH-$CH_2$-,

(iv) $-CH_2-CH=CH-$,

(v) $-CH_2-O-CH_2-$,

(vi) $-O-CH_2-$,

(vii) $-C\equiv C-$

or

(viii) -C=C- (trans)].

More preferred are those represented by the following Formula (III):

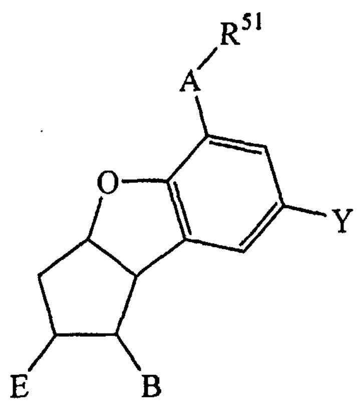

( III )

[wherein $R^{51}$ is

(i) $-COOR^{52}$

wherein $R^{52}$ is hydrogen, a pharmacologically acceptable cation or methyl, or

(ii)

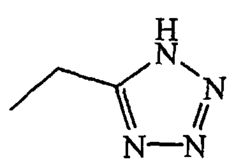

wherein Y is hydrogen or fluorine,
B is

(i)

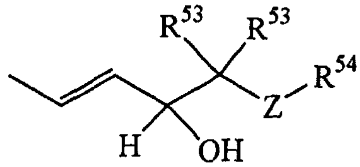

wherein $R^{53}$ is hydrogen, fluorine or $C_1$-$C_4$ alkyl, the two $R^{53}$s may be the same or different, Z represents the following in the definition of claim 2, $R^{54}$ is $C_5$-$C_7$ cycloalkyl, phenyl, or substituted phenyl (wherein the substituent is the same as the substituent defined for the substituted phenyl in claim 2), or

(ii)

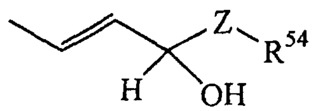

wherein Z and $R^{54}$ are defined as the same as the above,
E is hydrogen or -OH,
A is

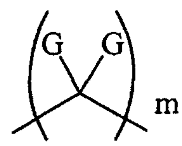

wherein m is an integer of 0 to 2, G represents hydrogen or fluorine, and all Gs may be the same or different].

Still more preferred are those represented by the following Formula (IV):

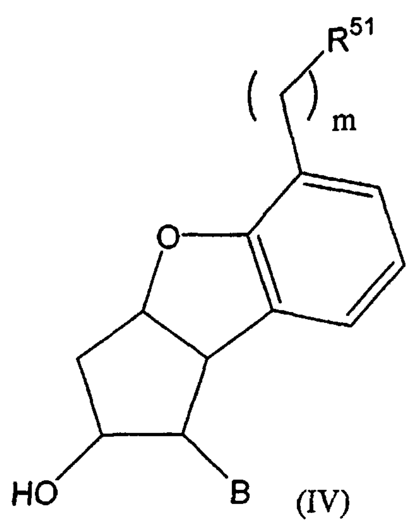

(IV)

[wherein $R^{51}$ is the same meaning as described above,
B is

(i)

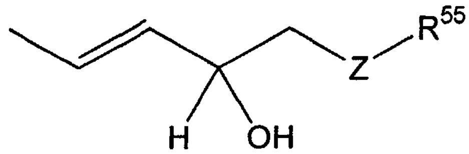

wherein Z is the following in the definition of claim 2, $R^{55}$ is $C_5$-$C_7$ cycloalkyl or phenyl, or

(ii)

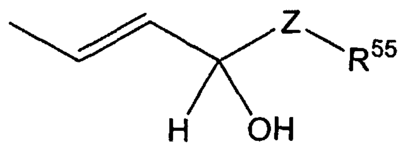

wherein Z and $R^{55}$ are defined as the same as the above, m represents an integer of 0 to 2].

Among these, especially preferred are the compounds represented by the following Formula (V) or (VI):

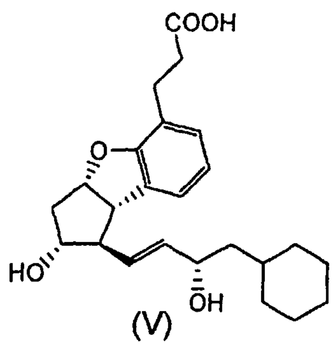

(V)

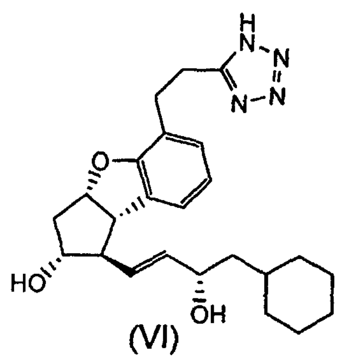

(VI)

Examples of the prostaglandin EP4 ligand of the present invention also include 3,7-dithiaprostanoic acid derivatives and azole derivatives. Examples of these include 3,7-dithiaprostanoic acid derivatives of the following Formula (VII), mixtures thereof with 8-epi compounds which are equilibrium compounds thereof, pharmacologically acceptable salts thereof, and clathrate compounds thereof included in cyclodextrin:

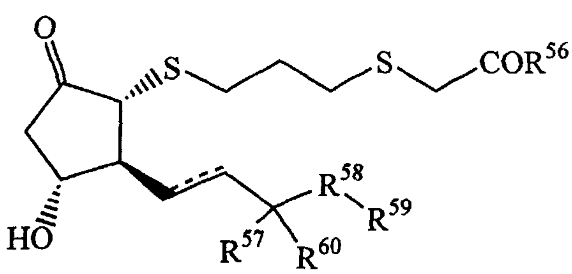

(VII )

[wherein $R^{56}$ is hydroxy, $C_1$-$C_6$ alkyloxy or $NR^{61}R^{62}$ (wherein $R^{61}$ and $R^{62}$ independently represent hydrogen or $C_1$-$C_6$

alkyl),
$R^{57}$ is hydrogen or hydroxy,
$R^{58}$ is a single bond or $C_1$-$C_6$ alkylene,
$R^{59}$ is

(I) $C_1$-$C_8$ alkyl, $C_2$-$C_8$ alkenyl or $C_2$-$C_8$ alkynyl,
(2) $C_1$-$C_8$ alkyl, $C_2$-$C_8$ alkenyl or $C_2$-$C_8$ alkynyl, each of which is substituted with 1 to 3 $C_1$-$C_6$ alkyloxy or halogen,
(3) $C_1$-$C_8$ alkyl, $C_2$-$C_8$ alkenyl or $C_2$-$C_8$ alkynyl, each of which is substituted with phenyl or $C_3$-$C_7$ cycloalkyl,
(4) phenyl, phenyloxy, $C_3$-$C_7$ cycloalkyl or $C_3$-$C_7$ cycloalkyloxy,
(5) furyl, furyloxy, thienyl, thienyloxy, naphthyl, naphthyloxy, phthalanyl or phthalanyloxy,
(6) phenyl, phenyloxy, $C_3$-$C_7$ cycloalkyl or $C_3$-$C_7$ cycloalkyloxy, each of which is substituted with 1 to 3 of the following groups:

$C_1$-$C_6$ alkyl, $C_2$-$C_6$ alkenyl, $C_2$-$C_6$ alkynyl, $C_1$-$C_6$ alkyloxy, $C_1$-$C_6$ alkyl substituted with $C_1$-$C_6$ alkyloxy, $C_1$-$C_6$ alkyloxy substituted with $C_1$-$C_6$ alkyloxy, $C_1$-$C_6$ alkyl substituted with $C_2$-$C_6$ alkenyloxy, $C_1$-$C_6$ alkyl substituted with 1 to 3 hydroxy, $C_1$-$C_6$ alkyl substituted with 1 to 3 halogen, $C_1$-$C_6$ alkylthio, $C_1$-$C_6$ alkyl substituted with $C_1$-$C_6$ alkylthio, $C_1$-$C_6$ alkyloxy substituted with $C_1$-$C_6$ alkylthio, $C_1$-$C_6$ alkyl substituted with $C_2$-$C_6$ alkenylthio, $C_1$-$C_6$ alkylsulfonyl, halogen, trihalomethyl, cyano, nitro, amino, hydroxy, $C_3$-$C_7$ cycloalkyl, $C_3$-$C_7$ cycloalkyloxy, $C_1$-$C_6$ alkyl substituted with $C_3$-$C_7$ cycloalkyl, $C_1$-$C_6$ alkyl substituted with $C_3$-$C_7$ cycloalkyloxy, phenyl, phenyloxy, $C_1$-$C_6$ alkyl substituted with phenyl, $C_2$-$C_6$ alkenyl substituted with phenyl, $C_2$-$C_6$ alkynyl substituted with phenyl, $C_1$-$C_6$ alkyl substituted with phenyloxy, $C_2$-$C_6$ alkenyl substituted with phenyloxy, $C_2$-$C_6$ alkynyl substituted with phenyloxy, furyl, furyloxy, $C_1$-$C_6$ alkyl substituted with furyl, $C_1$-$C_6$ alkyl substituted with furyloxy, thienyl, thienyloxy, $C_1$-$C_6$ alkyl substituted with thienyl, or $C_1$-$C_6$ alkyl substituted with thienyloxy (provided that the above-mentioned phenyl, furyl, thienyl and cycloalkyl may be substiuted with 1 to 3 $C_1$-$C_6$ alkyl, $C_1$-$C_6$ alkyloxy, $C_1$-$C_6$ alkyl substituted with $C_1$-$C_6$ alkyloxy, nitro, halogen, trihalomethyl, amino, or hydroxy), or

(7) furyl, furyloxy, thienyl, thienyloxy, naphthyl, naphthyloxy, phthalanyl or phthalanyloxy substituted with 1 to 3 of the following groups:

$C_1$-$C_6$ alkyl, $C_2$-$C_6$ alkenyl, $C_2C_6$ alkynyl, $C_1$-$C_6$ alkyloxy, $C_1$-$C_6$ alkyl substituted with $C_1$-$C_6$ alkyloxy, $C_1$-$C_6$ alkyloxy substituted with $C_1$-$C_6$ alkyloxy, $C_1$-$C_6$ alkyl substituted with $C_2$-$C_6$ alkenyloxy, $C_1$-$C_6$ alkyl substituted with 1 to 3 hydroxy, $C_1$-$C_6$ alkyl substituted with 1 to 3 halogen, $C_1$-$C_6$ alkylthio, $C_1$-$C_6$ alkyl substituted with $C_1$-$C_6$ alkylthio, $C_1$-$C_6$ alkyloxy substituted with $C_1$-$C_6$ alkylthio, $C_1$-$C_6$ alkyl substituted with $C_2$-$C_6$ alkenylthio, $C_1$-$C_6$ alkylsulfonyl, halogen, trihalomethyl, cyano, nitro, amino, hydroxy, $C_3$-$C_7$ cycloalkyl, $C_3$-$C_7$ cycloalkyloxy, $C_1$-$C_6$ alkyl substituted with $C_3$-$C_7$ cycloalkyl, $C_1$-$C_6$ alkyl substituted with $C_3$-$C_7$ cycloalkyloxy, phenyl, phenyloxy, $C_1$-$C_6$ alkyl substituted with phenyl, $C_2$-$C_6$ alkenyl substituted with phenyl, $C_2C_6$ alkynyl substituted with phenyl, $C_1$-$C_6$ alkyl substituted with phenyloxy, $C_2$-$C_6$ alkenyl substituted with phenyloxy, $C_2$-$C_6$ alkynyl substituted with phenyloxy, furyl, furyloxy, $C_1$-$C_6$ alkyl substituted with furyl, $C_1$-$C_6$ alkyl substituted with furyloxy, thienyl, thienyloxy, $C_1$-$C_6$ alkyl substituted with thienyl, or $C_1$-$C_6$ alkyl substituted with thienyloxy (provided that the above-mentioned phenyl, furyl, thienyl and cycloalkyl may be substiuted with 1 to 3 $C_1$-$C_6$ alkyl, $C_1$-$C_6$ alkyloxy, $C_1$-$C_6$ alkyl substituted with $C_1$-$C_6$ alkyloxy, nitro, halogen, trihalomethyl, amino, or hydroxy),

$R^{60}$ is hydrogen or $C_1$-$C_6$ alkyl,
the symbol
- - - - is double bond or single bond, with the proviso that when $R^{57}$ is hydrogen, the $C_1$-$C_6$ alkylene represented by $R^{58}$ may be substituted with one hydroxy], and also include the azole derivatives of the following Formula (VIII) and pharmacologically acceptable salts thereof:

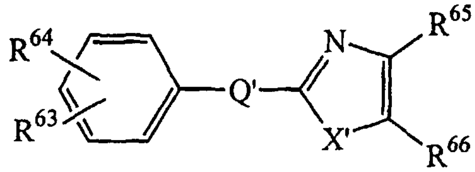

( VIII )

[wherein $R^{63}$ is

(1) $C_1$-$C_6$ alkyl substituted with hydroxy, protected carboxy or carboxy,
(2) carboxy,
(3) protected carboxy,
(4) carbamoyl,
(5) heterocyclic group,
(6) cyano,
(7) $C_1$-$C_6$ haloalkylsulfonyloxy,
(8) $C_1$-$C_6$ alkyloxy substituted with hydroxy or carbamoyl,
(9) aryl substituted with carboxy, protected carboxy, carbamoyl or heterocyclic group,
(10) amino which may be substituted with protected carboxy or $C_1$-$C_6$ alkylsulfonyl,

$R^{64}$ represents hydrogen or $C_1$-$C_6$ alkyl,
$R^{65}$ is aryl which may be substituted with halogen,
$R^{66}$ is aryl which may be substituted with halogen,
Q is

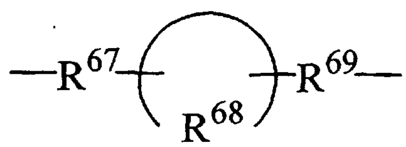

(wherein -$R^{67}$- is single bond or $C_1$-$C_6$ alkylene,

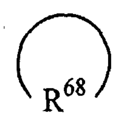

is $C_5$-$C_9$ cycloalkene, $C_3$-$C_9$ cycloalkane, $C_6$-$C_9$ bicycloalkene or $C_5$-$C_9$ bicycloalkane,
-$R^{69}$- is single bond or $C_1$-$C_6$ alkylene), and
X' is O, NH or S].

Here, the term "heterocyclic group" means saturated or unsaturated monocyclic or polycyclic group having at least one heteroatom selected from the group consisting of nitrogen, sulfur or oxygen; the term "protected carboxy" means (1) $C_1$-$C_6$ alkyl esters, (2) $C_2$-$C_6$ alkenyl esters, (3) $C_2$-$C_6$ alkynyl esters, (4) arylalkyl esters substituted with $C_1$-$C_6$ alkyl, $C_1$-$C_6$ alkyloxy, phenyl, nitro or halogen, or (5) aryl esters substituted with $C_1$-$C_6$ alkyl, $C_1$-$C_6$ alkyloxy, phenyl, nitro or halogen; and the term "aryl" means phenyl or naphthyl, which may be substituted with $C_1$-$C_6$ alkyl.

Among the compounds having binding activities for prostaglandin EP4 receptor, those having (an) asymmetric carbon(s) include various optical isomers, and those having at least two asymmetrical carbons include various diasteromers. The present invention include these optical isomers and respective isomers thereof. The present invention also include stereoisomers.

The compounds having binding activities for prostaglandin EP4 receptor and salts thereof *per se* are known, and may be produced by the methods described in the above-mentioned Laid-open patent application, International patent publications WO/8903387 and WO/0024727.

Examples of the pharmacologically acceptable salts include alkaline metal salts such as sodium salt and potassium salt; alkaline earth metal salts such as calcium salt and magnesium salt; amine salts such as methylamine salt, dimethylamine salt, trimethylamine salt, methylpiperidine salt, ethanolamine salt, diethanolamine salt, triethanolamine salt and lysine salt; ammonium salt; basic amino acid salts; inorganic acid salts such as hydrochloric acid salt and sulfuric acid salt; organic carboxylic acid salts such as acetic acid salt, tartaric acid salt and maleic acid salt; and organic sulfonic acid salts such as p-toluenesulfonic acid salt. However, the pharmacologically acceptable salts are not restricted to those described above.

In application of the agent for modulating growth or generation of hair according to the present invention, one or several prostaglandin EP4 receptor ligands or (a) salt(s) thereof may be applied to head as it is, or the ligand(s) may be applied after being admixed with a vehicle, stablizer and/or the like, which are usually used in formulation of pharmaceuticals. Examples of such an additive include animal oils; plant oils; paraffin; gum arabic; saccharides such

as starch, lactose, sucrose, glucose, dextrin and mannitol; inorganic acid salts such as calcium carbonate and calcium sulfate; organic acid salts such as sodium citrate, sodium lactate and magnesium stearate; water-soluble polymers such as methylcellulose, gelatin, polyethylene glycol, polyvinyl alcohol, polyvinylpyrrolidone, hydroxyethylcellulose and hydroxypropylcellulose; alcohols such as ethanol, glycerin, propylene glycol and sorbitol; and surfactants such as sorbitan aliphatic acid esters, polyoxyethylenesorbitan aliphatic acid esters, polyoxyethylene aliphatic acid esters and glycerin aliphatic acid esters. However, the additives are not restricted to those described above.

The agent for modulating growth or generation of hair according to the present invention may be applied in various dosage forms. More particularly, the agent may be in a form which is usually used, such as in the form of liquid, jelly, emulsion, aerosol or ointment. For example, it may be used as a hair-growing agent, hair tonic, hair liquid, hair lotion, hair cream, hair gel, hair foam, hair mist, hair oil, hair treatment, mousse, shampoo or rinse. Alternatively, it may be used as an injection solution for subcutaneous, intravenous or local injections. Further, it may be formulated into a dosage form for oral administration, such as tablets, powder, granules, balls and capsules. However, the formulations are not restricted to those described above.

As will be concretely shown in the Examples below, since the agent for modulating growth or generation of hair according to the present invention has a high selectivity to EP4 receptor subtype and exhibits hair-growing or hair-generating activities to epilated rabbit models of different ages (17 and 8 weeks old), it may be used for therapy or treatment of, for example, atrichia and hirsutism, or alopecia (male pattern alopecia and alopecia areata) as abnormal hair growth.

The dose of administration of the agent for modulating growth or generation of hair according to the present invention varies depending on the symptom, age, individual difference, formulation of the agent and the like, and usually the agent may be administered in an amount of 0.0001 mg to 1000 mg, preferably 0.001 mg to 100 mg in one time or in several times. However, the dose of administration is not restricted thereto.

The agent for modulating growth or generation of hair according to the present invention may be administered together with (a) hair-growing agent(s) (blood circulation promoters, potassium channel openers, androgen inhibitors, antiinflammatory agents, follicle-stimulators, antioxidants and keratolytic drugs) other than the agent of the present invention simultaneously or with a time interval. Examples of the hair-growing agents which may be used together with the agent for modulating growth or generation of hair according to the present invention include blood circulation promoters such as carpronium hydrochloride, cepharanthin and nicotinamide; potassium channel openers such as minoxidil, pinacidil and carpronium hydrochloride; androgen inhibitors such as 5α reductase inhibitors such as finasteride, and eugenyl glucoside; antiinflammatory agents such as glycyrrhizic acid and derivatives thereof, and glycyrrhetinic acid and derivatives thereof; follicle stimulators such as pentadecanoic acid glyceride, carpronium hydrochloride and procyanidin B; antioxidants such as vitamins including vitamin C, vitamin E and derivatives thereof; keratolytic agents such as aspirin; hormones such as estrogen and adenocorticotropic hormone; and amino acids such as taurine.

The agent for modulating growth or generation of hair according to the present invention may also be administered to animals other than human. That is, the agent can modulate growth or generation of hair by administering it to animals other than human, so that it can be applied for epilation and abnormality of fur, or therapy or treatment of animals other than human.

Examples

To describe the present invention in more detail, examples thereof will now be described.

<u>Example 1</u>

Receptor Binding Experiment Using $PGE_2$ Receptor Subtype-expressing Cells

The compound of the Formula (I) described above was tested for its selective binding activity to EP4 receptor by using $PGE_2$ receptor subtype-expressing cells.

According to the reported method (Breyer,R.M. et al.,J.Biol.Chem.269,6163-6169(1994)), cloned human $PGE_2$ receptor subtypes (EP3 and EP4) were transiently expressed by COS-7 cells. EP2 was stably expressed by CHO cells. These cells were prepared into membrane specimens. Each of the prepared membrane fractions (10 μg/tube) was incubated with a reaction solution containing $^3H$-$PGE_2$ at 30°C for 1 hour. The reaction was terminated by iced buffer (l0mM MES(pH6.0), l0mM $MgC_{l2}$, 1mM EDTA), and the resultant was subjected to filtration by means of suction under reduced pressure to trap the bound $^3H$-$PGE_2$ on a glass filter (GF/C), followed by measurement of the radioactivity with a liquid scintillation counter.

The Kd value was determined from Scatchard plot according to the conventional method. Non-specific binding was determined in terms of the amount of bound radioactivity in the presence of an excess amount (5 μM) of non-labeled $PGE_2$. Measurement of inhibition of $^3H$-$PGE_2$ binding by Compound I was carried out by adding $^3H$-$PGE_2$ (5

nM) and various concentrations of Compound 1 of the following Formula (V):

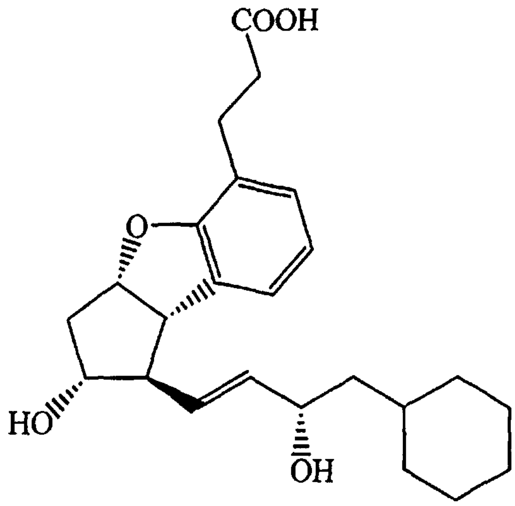

(V)

Compound 1 was prepared according to Example 17 of Japanese Patent No. 1933167.
The dissociation constant Ki (nM) of Compound 1 was calculated according to the following equation:

$$Ki=IC_{50}/(1+([C]/Kd))$$

As a result, the dissociation constant Ki of Compound 1 to EP4 was 19 nM. Thus, Compound 1 has a high selectivity to EP4 receptor subtype and its binding abilities to other receptor subtypes are low, so that it is thought that its side effect is small when it is administered.

Example 2

Hair-growing or Hair-generating Action

To investigate the hair-growth or hair-generation modulation action of Compound 1 which was shown to be a prostaglandin EP4 receptor agonist in Example 1, hair-growth/generation test was performed using rabbits. The test was carried out as follows. The hair on the back of each rabbit was removed with an electric clipper and evacream was applied for a short time to the back so as to epilate the back. After wiping the epilated region with water, test compound was subcutaneously administered to the back at a dose of 0.25 ml/kg (dissolved in 0.9% sodium chloride solution) once a day from the second day to 20th day after epilation. The state of generation of hair was observed and the length of the hair was measured.
As a result, generation of hair was observed on the 15th day after administration of Compound 1, and the hair-generated area became not less than 50% in 3 rabbits out of 4 rabbits on the 20th day after administration. Further, the length of the new hair was increased by administration of Compound 1. The results are shown in Table 1. From these results, it was proved that Compound 1 has excellent hair-growing or hair-genrating activity.

Table 1

| Hair-Growth or Hair-Generation Modulation Activity of Compound 1 (20th day) | | | |
|---|---|---|---|
| | Rate of Hair-generated Area | | Length of New Hair |
| | (%) | Rate of Rabbits with 50% or More Hair-Generated Area | (mm) |
| Control Group | 10.0±5.5 | 0/4 | 4.8+0.4 |

Table 1 (continued)

| Hair-Growth or Hair-Generation Modulation Activity of Compound 1 (20th day) | | | |
|---|---|---|---|
| | Rate of Hair-generated Area | | Length of New Hair |
| | (%) | Rate of Rabbits with 50% or More Hair-Generated Area | (mm) |
| Compound 1-administered Group | | | |
| 0.03mg/kg | 15.8±13.2 | 1/4 | 6.0±0.6 |
| 0.1mg/kg | 48.8±13.6 | 3/4 | 7.3+0.5* |
| Values indicate mean ± standard error. | | | |

*: p<0.05 vs. control group (Dunnett's t-test)

Example 3

Magnus Experiment Using Rabbit Saphenous Vein

Investigation of agonistic activity to EP4 was carried out by an improved method of the method of Lydford et al. (S. J. Lydford et al., Br. J. Pharmacol. 117, 13-20 (1996)) using rabbit saphenous veins. That is, each rabbit was deeply anesthetized by administering pentobarbital (50 mg/kg) to auricular vein, and then sacrificed by bleeding by cutting carotid artery. Saphenous vein was carefully but quickly removed and transferred into Krebs solution (NaCl 118.1 mM, KCl 5.31 mM, $MgSO_4$ 1.01 mM, $CaCl_2$ 3.52 mM, $NaH_2PO_4$ 1.09 mM, $NaHCO_3$ 25.0 mM, glucose 9.99 mM, pH 7.4) which was preliminarily saturated with 95% $O_2$ + 5% $CO_2$ mixed gas. The connective tissue and the fatty tissue of each saphenous vein were isolated using a scissors for ophthalmology to prepare a ring preparation with a width of about 4 mm. This ring preparation was mounted on a hook for blood vessels, and then suspended *in* a Magnus tube filled with Krebs solution aerated with 95% $O_2$ + 5% $CO_2$ mixed gas and kept at 37°C. The isometric tension of each blood vessel was recorded on a recorder through an isometric transducer. To each blood vessel, a base tension of 1.0 g was applied and the blood vessel tissue was stabilized for about 30 minutes until the baseline became stabilized. After the baseline became stable, the blood vessel was contracted with 40 mM KCl, and the shrinkage and washing were repeated until the contraction became stable. After the contraction with KCl became stable, a thromboxane antagonist S-145 (1000 nM) was applied previously and then cumulative application experiments of Compounds 1 to 7 were carried out. More specifically, the reactivity to $PGE_2$ which was a positive control drug was measured, and after washing, the action of each compound was studied. The effectiveness of each drug was expressed in terms of the concentration ($EC_{50}$) at which the rate of relaxation of the test drug to the contraction by 40 mM KCl was *50%.* $EC_{5o}$ value was calculated by plotting logarithm of the concentration of each drug along the abscissa and plotting the rate of relaxation with respect to the contraction by 40 mM KCl along the ordinate, and by determining the concentration at which the height of contraction was 50% based on the linear area of the concentration-reaction curve in each experiment.

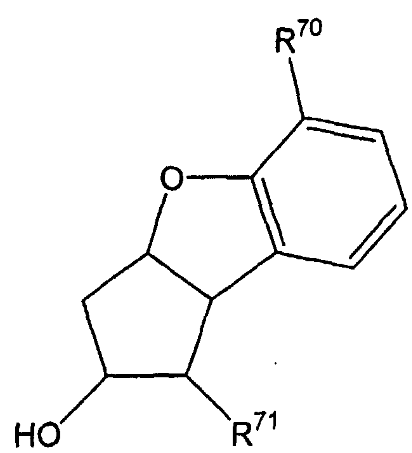

Compounds 2, 6 and 7 were prepared as described in International Publication WO/0024727, Compound 3

was prepared as described in Japanese Patent No. 2893812, Compound 4 was prepared as described in Japanese Patent No. 1933167, and Compound 5 was prepared as described in Japanese Patent No. 1974492.

The results are shown in Table 2. Thus, Compounds 1 to 7 have high selectivities to EP4 receptor subtype and their binding abilities to other receptor subtypes are low, so that that their side effects are thought to be small when they are administered.

Table 2 Agonistic Activity of Compounds to EP4 Receptor in Rabbit Saphenous Vein Relaxation Reaction

| Compound | $R^{70}$ | $R^{71}$ | $EC_{50}$ (nM) |
|---|---|---|---|
| 1 | 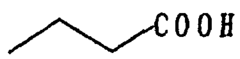 | 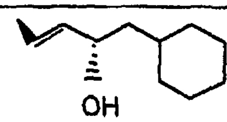 | 6 |
| 2 | 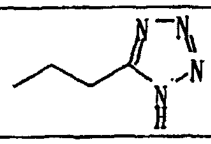 | 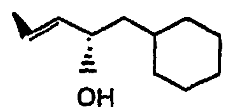 | 3 |
| 3 | 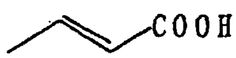 | 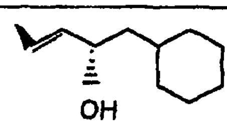 | 6 |
| 4 | 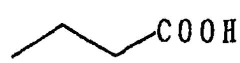 | 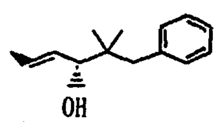 | 7 |
| 5 | 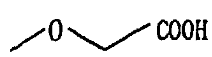 | 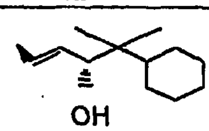 | 26 |
| 6 | 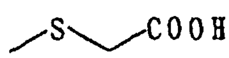 | 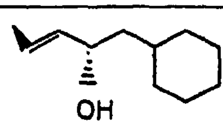 | 33 |
| 7 | 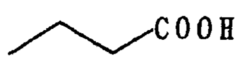 | 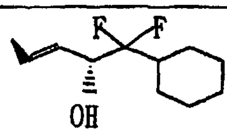 | 2 |

Example 4

Hair-growing or Hair-generating Action

To investigate the hair-growth or hair-generation modulation action of Compound 2 which was shown to be a prostaglandin EP4 receptor agonist in Example 3, hair-growth/generation test was performed using young rabbits (New Zealand White rabbits, male, 8 weeks old, Kitayama Labes). The test was carried out as follows. The hair on the back of each rabbit was removed and evacream was applied for a short time to the back so as to epilate the back. After wiping the epilated region with water, test compound was subcutaneously administered to the back at a dose of 0.25 ml/kg once a day from the second day to 7th day after epilation. The length of the hair was measured.

As a result, the length of the new hair was increased. The results are shown in Table 2. By these results, it was proved that Compound 2 has an excellent hair-growing or hair-generating activity.

Table 3

| Hair-Growth or Hair-Generation Modulation Activity of Compound 1 (7th day) | |
|---|---|
| | Length of New Hair (mm) |
| Control Group | 9.2±0.3 |
| Compound 2-administered Group 0.03mg/kg | 11.7±0.5* |
| Values indicate mean ± standard error. | |

*: $p<0.05$ vs. control group (Student's t-test)

Industrial Availability

The agent for modulating growth or generation of hair according to the present invention has an excellent activity to modulate growth or generation of hair, and its side effects are small. Therefore, it is useful for therapy or treatment of atrichia and hirsutism, or alopecia (male pattern alopecia and alopecia areata) as abnormal hair growth.

## Claims

1. An agent for modulating growth or generation of hair comprising a prostaglandin EP4 ligand as an active ingredient.

2. The agent for modulating growth or generation of hair according to claim 1, wherein the said prostaglandin EP4 receptor ligand is a 5,6,7-trinor-4,8-inter-m-phenylene $PGI_2$ derivative of the following Formula (I) or a pharmacologically acceptable salt thereof:

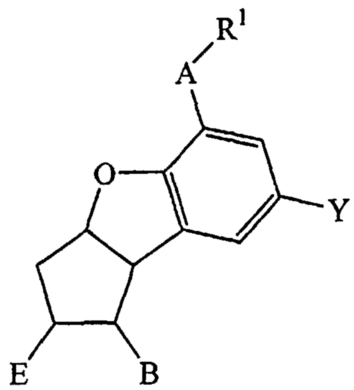

(I)

[wherein
$R^1$ is

(i)

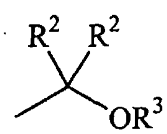

wherein $R^2$ is hydrogen, $C_1$-$C_4$ linear alkyl, $C_3$ or $C_4$ branched alkyl, trifluoromethyl, -C(=O)-$R^4$, or -C(=O)-O-$R^4$, wherein $R^4$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted

phenyl (wherein the substituent is at least one fluorine, chlorine, bromine, iodine, trifluoromethyl, $C_1$-$C_4$ alkyl, nitro, cyano, methoxy, phenyl, phenoxy, p-acetamidebenzamide, -CH=N-NH-C(=O)-$NH_2$, -NH-C(=O)-Ph, -NH-C(=O)-$CH_3$ or
-NH-C(=O)-$NH_2$), and the two $R^2$s may be the same or different; $R^3$ is hydrogen, $C_1$-$C_4$ alkyl, $C_1$-$C_{12}$ acyl, $C_7$-$C_{16}$ aroyl, $C_7$-$C_{16}$ aralkyl, tetrahydropyranyl, tetrahydrofuranyl, 1-ethoxyethyl, allyl, tert-butyl or tert-butyldimethylsilyl,

(ii) -$COOR^5$

wherein $R^5$ is
(1) hydrogen or pharmacologically acceptable cation,
(2) $C_1$-$C_{12}$ linear alkyl or $C_3$-$C_{14}$ branched alkyl,

(3) -Z-$R^6$

wherein Z is a valence bond, or linear or branched alkylene represented by the formula $C_tH_{2t}$ wherein t represents an integer of 1 to 6, $R^6$ is $C_3$-$C_{12}$ cycloalkyl, or $C_3$-$C_{12}$ cycloalkyl substituted with 1 to 4 $R^7$s wherein $R^7$ is hydrogen or $C_1$-$C_5$ alkyl,

(4) $-(CH_2CH_2O)_nCH_3$

wherein n represents an integer of 1 to 5,

(5) -Z-Ar

wherein Z is defined as the same as the above, Ar is phenyl, α-naphthyl, β-naphthyl, 2-pyridyl, 3-pyridyl, 4-pyridyl, α-furyl, β-furyl, α-thienyl, β-thienyl or substituted phenyl (wherein the substituent is the same as the substituent defined for the substituted phenyl mentioned above),

(6) $-C_tH_{2t}COOR^8$

wherein t is defined as the same as the above, $R^8$ is hydrogen or $C_1$-$C_5$ alkyl,

(7) $-C_tH_{2t}N(R^9)_2$

wherein t is defined as the same as above, $R^9$ is hydrogen or $C_1$-$C_5$ alkyl, and the two $R^9$s may be the same or different,

(8) -CH($R^{10}$)-C(=O)-R11

wherein $R^{10}$ is hydrogen or benzoyl, $R^{11}$ is phenyl, p-bromophenyl, p-chlorophenyl, p-biphenyl, p-nitrophenyl, p-benzamidephenyl or 2-naphthyl,

(9) $-C_pH_{2p}$-W-$_R{}^{12}$

wherein p represents an integer of 1 to 5, W is -CH=CH-, -CH=C($R^{13}$)- or
-C≡C- wherein $R^{13}$ is $C_1$-$C_{30}$ linear alkyl, $C_3$-$C_{30}$ branched alkyl or $C_7$-$C_{30}$ aralkyl, $R^{12}$ is hydrogen, $C_1$-$C_{30}$ linear alkyl, $C_3$-$C_{30}$ branched alkyl or $C_7$-$C_{30}$ aralkyl, or (10) $-CH(CH_2OR^{14})_2$
wherein $R^{14}$ is $C_1$-$C_{30}$ alkyl or $C_1$-$C_{30}$ acyl, and the two $R^{14}$s may be the same or different,

(iii)

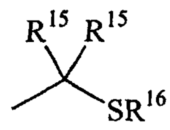

wherein $R^{15}$ represents is hydrogen, $C_1$-$C_4$ linear alkyl, $C_3$ or $C_4$ branched alkyl, trifluoromethyl, -C(=O)-$R^{17}$ -C(=O)-O-$R^{17}$ wherein $R^{17}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above), and the two $R^{15}$s may be the same or different; $R^{16}$ is hydrogen, $C_1$-$C_{12}$ linear alkyl, $C_3$-$C_{14}$ branched alkyl, phenyl or substituted phenyl (wherein the substituent is the same as the substituent defined for the substituted phenyl mentioned above), or
-C(=O)-$R^{18}$ wherein $R^{18}$ represents $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above),

(iv) $-CH_2-R^{19}$

wherein $R^{19}$ is

(1)

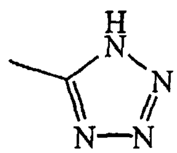

(2)

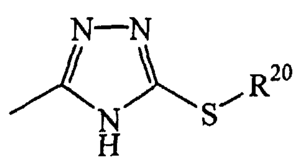

wherein $R^{20}$ represents hydrogen, $C_1$-$C_{12}$ linear alkyl, $C_3$-$C_{14}$ branched alkyl, phenyl, substituted phenyl (wherein the substituent is the same as the substituent defined for the substituted phenyl mentioned above), or -C(=O)-$R^{21}$ wherein $R^{21}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl, or substituted phenyl (wherein the substituent is the same as the substituent defined for the substituted phenyl mentioned above),

(3)

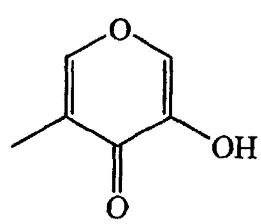

(4)

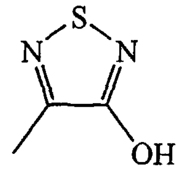

(5)

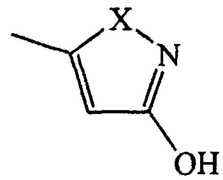

wherein X represents -O- or -S-, or
(6) azide,

(v) $-C(R^{22})^3$

wherein $R^{22}$ represents hydrogen, fluorine, chlorine, bromine, iodine, cyano or $C_1$-$C_4$ alkyl, and all of the $R^{22}$s may be the same or different,

(vi)

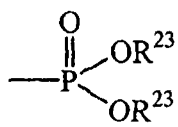

wherein $R^{23}$ represents hydrogen, $C_1$-$C_4$ alkyl, phenyl, substituted phenyl (wherein the substituent is the same as the substituent defined for the substituted phenyl mentioned above), $-CH_2-OR^{24}$ (wherein $R^{24}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl, or substituted phenyl (wherein the substituent is the same as the substituent defined for the substituted phenyl mentioned above), or pharmacologically acceptable cation, and the two $R^{23}$s may be the same or different,

(vii) $-N(R^{25})_2$

wherein $R^{25}$ is hydrogen, $C_1$-$C_{12}$ linear alkyl, $C_3$-$C_{14}$ branched alkyl, $C_3$-$C_{12}$ cycloalkyl, $C_4$-$C_{13}$ cycloalkylalkyl, $C_7$-$C_{12}$ aralkyl, $-C(=O)-R^{26}$,$-C(=O)-O-R^{26}$, $-SO_2-R^{26}$, phenyl or substituted phenyl (wherein the substituent is the same as the substituent defined for the substituted phenyl mentioned above), $R^{26}$ is $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above), the two $R^{25}$s may be the same or different (when one of the $R^{25}$s is -SO2-$R^{26}$, the other $R^{25}$ is not $-SO_2-R^{26}$),

(viii) $-(C(=O)CH_2)_k-H$

wherein k is an integer of 1 or 2, or

(ix) $-C(=O)-N(R^{27})_2$

wherein $R^{27}$ is hydrogen, $C_1$-$C_{12}$ alkyl, $C_3$-$C_{12}$ cycloalkyl, phenyl, substituted phenyl (wherein the substituent is the same as the substituent defined for the substituted phenyl mentioned above), $C_4$-$C_{13}$ cycloalkylalkyl, $C_7$-$C_{12}$ aralkyl, cyano or $-SO_2-R^{28}$ wherein $R^{28}$ is $C_1$-$C_{12}$ alkyl, $C_3$-$C_{12}$ cycloalkyl, phenyl, substituted phenyl (wherein the substituent is the same as the substituent defined for the substituted phenyl mentioned above), $C_4$-$C_{13}$ cycloalkylalkyl, or $C_7$-$C_{12}$ aralkyl, and the two $R^{27}$s may be the same or different (when one of the $R^{27}$s is $-SO_2-R^{28}$, the other $R^{27}$ is not $-SO_2-R^{28}$);
Y is hydrogen, $C_1$-$C_4$ alkyl, fluorine, chlorine, bromine, formyl, methoxy or nitro;
B is

(i)

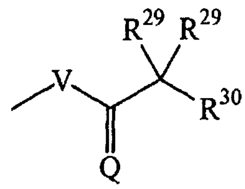

wherein V is

(1) $-CH_2CH_2-$,

(2) $-C{\equiv}C-$,

or

(3) $-CH=C(R^{31})-$

wherein $R^{31}$ is hydrogen, $C_1$-$C_5$ alkyl, fluorine, chlorine, bromine or iodine,
Q is

(1) =O

(2)

$—R^{32}$

$—OR^{33}$

or

(3) $—R^{32}$

$—R^{32}$

wherein $R^{32}$ is hydrogen, $C_1$-$C_4$ linear alkyl, $C_3$ or $C_4$ branched alkyl, trifluoromethyl, $-C(=O)-R^{34}$, or $-C(=O)-O-R^{34}$ wherein $R^{34}$ represents $C_1$-$C_{12}$ linear alkyl, $C_3$-$C_{14}$ branched alkyl, $C_3$-$C_{12}$ cycloalkyl, $C_7$-$C_{12}$ aralkyl, phenyl or substituted phenyl (wherein the substituent is the same as the substituent defined for the substituted phenyl mentioned above); $R^{33}$ is hydrogen, $C_1$-$C_4$ alkyl, $C_1$-$C_{12}$ acyl, $C_7$-$C_{16}$ aroyl, $C_7$-$C_{16}$ aralkyl, tetrahydropyranyl, tetrahydrofuranyl, 1-ethoxyethyl, allyl, tert-butyl or tert-butyldimethylsilyl, and the two $R^{32}$s may be the same or different;

$R^{29}$ is hydrogen, fluorine, chlorine, bromine, iodine, cyano or $C_1$-$C_4$ alkyl, and the two $R^{29}$s may be the same or different;
$R^{30}$ is

(1) $-Z-R^{35}$

wherein Z is defined as the same as the above, $R^{35}$ is $C_1$-$C_{12}$ linear alkyl, $C_3$-$C_{14}$ branched alkyl, $C_3$-$C_{12}$ cycloalkyl, $C_4$-$C_{13}$ cycloalkylalkyl, $C_3$-$C_{12}$ cycloalkyl substituted with 1 to 4 $R^{36}$s (wherein $R^{36}$ is hydrogen or $C_1$-$C_5$ alkyl), $C_4$-$C_{13}$ cycloalkylalkyl substituted with 1 to 3 $R^{36}$s (wherein $R^{36}$ is defined as the same as the above), phenyl, substituted phenyl (wherein the substituent is the same as the substituent defined for the substituted phenyl mentioned above), α-naphthyl, β-naphthyl, 2-pyridyl, 3-pyridyl, 4-pyridyl, α-furyl, β-furyl, α-thienyl or β-thienyl,

(2) $-Z-O-R^{35}$

wherein Z and $R^{35}$ are defined as the same as the above,

(3) $-Z-CH=C(R^{35})_2$

wherein Z and $R^{35}$ are defined as the same as the above, and the two $R^{35}$s may be the same or different, or

(4) $-Z-C{\equiv}C-R^{35}$

wherein Z and $R^{35}$ are defined as the same as the above,

(ii)

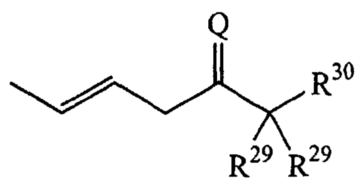

wherein Q, $R^{29}$ and $R^{30}$ are defined as the same as the above, and the two $R^{29}$s may be the same or different, or

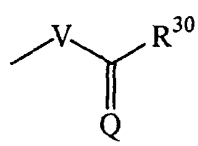

wherein V, Q and $R^{30}$ are defined as the same as the above;
E represents hydrogen or $-OR^{33}$ wherein $R^{33}$ is defined as the same as the above;
A is

(i)

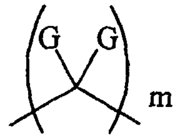

wherein m represents an integer of 0 to 5, G represents hydrogen, fluorine, chlorine, bromine, iodine, trifuluoromethyl, $C_1$-$C_4$ linear alkyl or $C_3$-$C_6$ branched alkyl, and all Gs may be the same or different,

(ii)

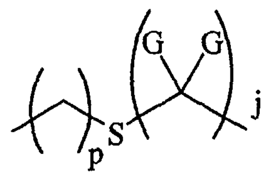

wherein j represents an integer of 1 to 4, p represents an integer of 0 or 1, G is defined as the same as the above, and all Gs may be the same or different,

(iii) $-CH=CH-CH_2-$,

(iv) $-CH_2-CH=CH-$,

(v) $-CH_2-O-CH_2-$,

(vi) $-O-CH_2-$,

(vii) $-C \equiv C-$,

or

(viii) -C=C- (trans)]

**3.** The agent for modulating growth or generation of hair according to claim 2, wherein the said 5,6,7-trinor-4,8-inter-m-phenylene PGI2 derivative is represented by the following Formula (I):

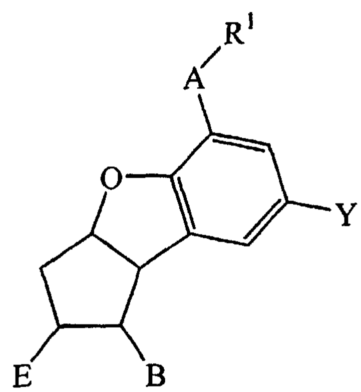

(I)

[wherein $R^1$, Y, E and A represent the following in the definition of claim 2, B is

(i)

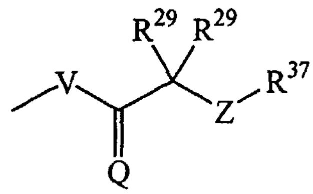

wherein V, Q, $R^{29}$ and Z represent the following in the definition of claim 2, the two $R^{29}$s may be the same or different, $R^{37}$ is $C_3$-$C_{12}$ cycloalkyl, $C_4$-$C_{13}$ cycloalkylalkyl, $C_3$-$C_{12}$ cycloalkyl substituted with 1 to 4 $R^{38}$s (wherein $R^{38}$ is hydrogen or $C_1$-$C_5$ alkyl), $C_4$-$C_{13}$ cycloalkylalkyl substituted with 1 to 3 $R^{38}$s (wherein $R^{38}$ is defined as the same as the above), phenyl, substituted phenyl (wherein the substituent is the same as the substituent defined for the substituted phenyl in claim 2), α-naphthyl, β-naphthyl, 2-pyridyl, 3-pyridyl, 4-pyridyl, α-furyl, β-furyl, α-thienyl or β-thienyl,

(ii)

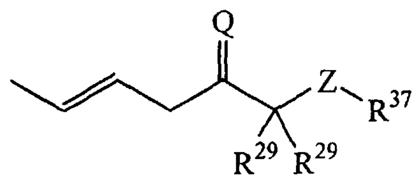

wherein Q, $R^{29}$, Z and $R^{37}$ are defined as the same as the above, and the two $R^{29}$s may be the same or different, or

(iii)

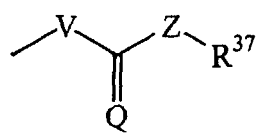

wherein V, Q, Z and $R^{37}$ are defined as the same as the above].

**4.** The agent for modulating growth or generation of hair according to claim 3, wherein the said 5,6,7-trinor-4,8-inter-m-phenylene PGI2 derivative is represented by the following Formula (II):

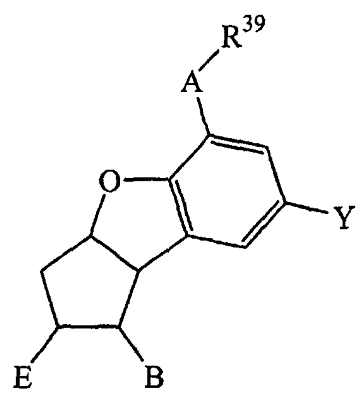

( II )

[wherein $R^{39}$ is

(i)

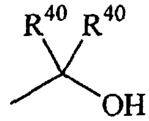

wherein $R^{40}$ is hydrogen, $C_1$-$C_4$ linear alkyl or trifluoromethyl, the two $R^{40}$ may be the same or different,

(ii) $-COOR^{41}$

wherein $R^{41}$ is hydrogen, a pharmacologically acceptable cation or $C_1$-$C_{12}$ linear alkyl,

(iii)

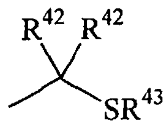

wherein $R^{42}$ is hydrogen, $C_1$-$C_4$ linear alkyl or trifluoromethyl, the two $R^{42}$s may be the same or different, $R^{43}$ is hydrogen, $C_1$-$C_4$ linear alkyl, phenyl, or $-C(=O)-R^{44}$
wherein $R^{44}$ represents $C_1$-$C_4$ linear alkyl,

(iv) $-CH_{2-}R^{45}$

wherein $R^{45}$ is

(1)

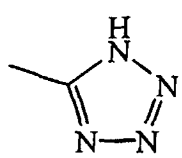

(2)

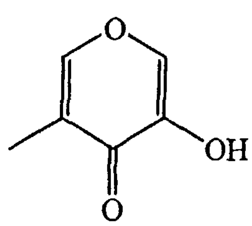

or

(3)

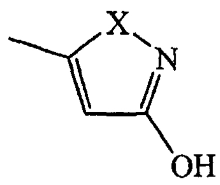

wherein X represents the following in the definition of claim 2,

(v) $-C(R^{46})_3$

wherein $R^{46}$ represents hydrogen, fluorine, cyano or $C_1$-$C_4$ alkyl, and all $R^{46}$s may be the same or different,

(vi)

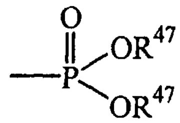

wherein $R^{47}$ represents hydrogen, $C_1$-$C_4$ alkyl, or a pharmacologically acceptable cation, and the two $R^{47}$s may be the same or different, or

(vii) $-N(R^{48})_2$

wherein $R^{48}$ is hydrogen, $-C(=O)-R^{49}$ or $-SO_2-R^{49}$ wherein $R^{49}$ is $C_1$-$C_4$ linear alkyl or phenyl, and the two $R^{48}$s may be the same or different (when one of $R^{48}$s is $-SO_2-R^{49}$, the other $R^{48}$ is not $-SO_2-R^{49}$),
Y is hydrogen, fluorine, chlorine or bromine,
B is

(i)

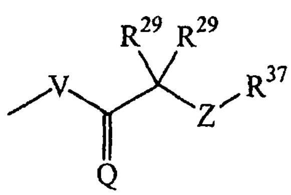

wherein V is

(1) $-CH_2CH_2-$,

(2) $-C{\equiv}C-$,

or

(3) $-CH{=}CH-$,

Q is

(1) =O,

(2)

—$R^{50}$

—$OR^{33}$

or

(3)

—$R^{50}$

—$R^{50}$

wherein $R^{50}$ is hydrogen, $C_1$-$C_4$ linear alkyl, $C_3$ or $C_4$ branched alkyl, or trifluoromethyl, $R^{33}$ represents the following in the definition of claim 2, the two $R^{50}$s may be the same or different, $R^{29}$ represents the following in the definition of claim 2, and the two $R^{29}$s may be the same or different, Z represents the following in the definition of claim 2, and $R^{37}$ represents the following in the definition of claim 3,

(ii)

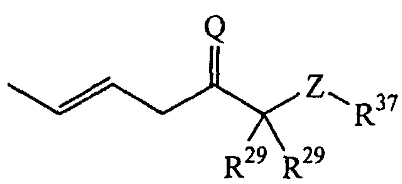

wherein Q, $R^{29}$, Z and $R^{37}$ are defined as the same as the above, and the two $R^{29}$s may be the same or different, or

(iii)

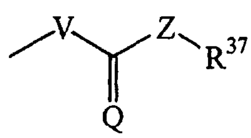

wherein V, Q, Z and $R^{37}$ are defined as the same as the above,
E represents the following in the definition of claim 2,
A is

(i)

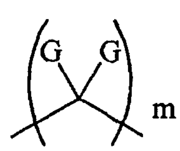

wherein m represents an integer of 0 to 3, G is hydrogen, fluorine, chlorine, bromine, iodine, trifluoromethyl or $C_1$-$C_4$ linear alkyl, and all Gs may be the same or different,

(ii)

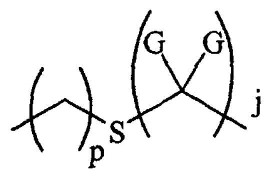

wherein j represents an integer of 1 or 2, p represents the following in the definition of claim 2, G is defined as the same as the above, and all Gs may be the same or different,

(iii) $-CH=CH-CH_2-$,

(iv) $-CH_2-CH=CH-$,

(v) $-CH_2-O-CH_2-$,

(vi) $-O-CH_2-$,

(vii) $-C\equiv C-$

or

(viii) -C=C- (trans)].

**5.** The agent for modulating growth or generation of hair according to claim 4, wherein the said 5,6,7-trinor-4,8-inter-m-phenylene PGI2 derivative is represented by the following Formula (III):

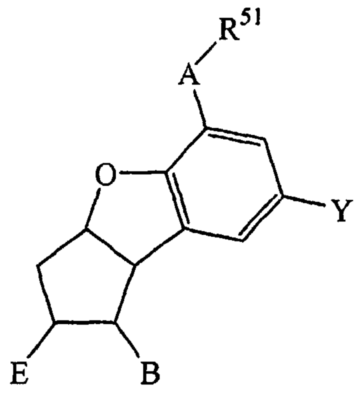

(III)

[wherein $R^{51}$ is

(i) -COOR$^{52}$

wherein $R^{52}$ is hydrogen, a pharmacologically acceptable cation or methyl, or

(ii)

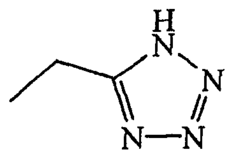

wherein Y is hydrogen or fluorine,
B is

(i)

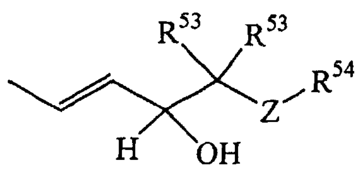

wherein $R^{53}$ is hydrogen, fluorine or $C_1$-$C_4$ alkyl, the two $R^{53}$s may be the same or different, Z represents the following in the definition of claim 2, $R^{54}$ is $C_5$-$C_7$ cycloalkyl, phenyl, or substituted phenyl (wherein the substituent is the same as the substituent defined for the substituted phenyl in claim 2), or

(ii)

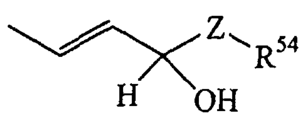

wherein Z and $R^{54}$ are defined as the same as the above,
E is hydrogen or -OH,
A is

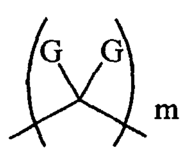

wherein m represents an integer of 0 to 2, G represents hydrogen or fluorine, and all Gs may be the same or different].

**6.** The agent for modulating growth or generation of hair according to claim 5, wherein the said 5,6,7-trinor-4,8-inter-m-phenylene PGI2 derivative is represented by the following Formula (IV):

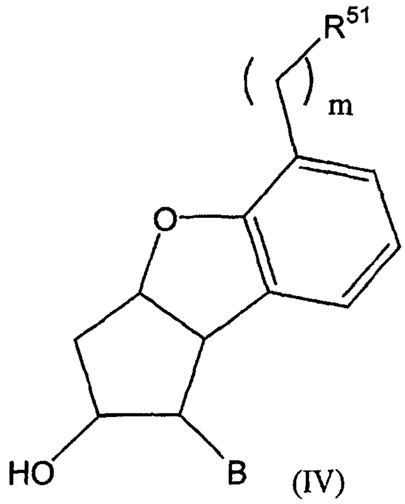

(IV)

[wherein $R^{51}$ represents the following in the definition of claim 5,
B is

(i)

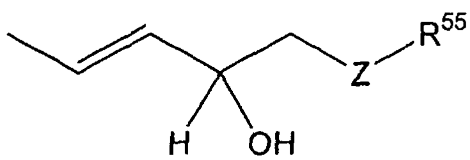

wherein Z represents the following in the definition of claim 2, $R^{55}$ is $C_5$-$C_7$ cycloalkyl or phenyl, or

(ii)

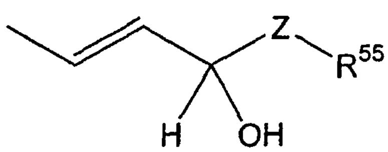

wherein Z and $R^{55}$ are defined as the same as the above, m represents an integer of 0 to 2].

**7.** The agent for modulating growth or generation of hair according to claim 6,
wherein the said 5,6,7-trinor-4,8-inter-m-phenylene $PGI_2$ derivative is represented by the following Formula (V) or (VI).

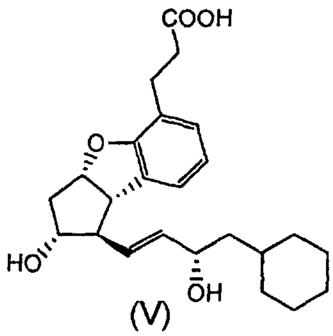

(V)

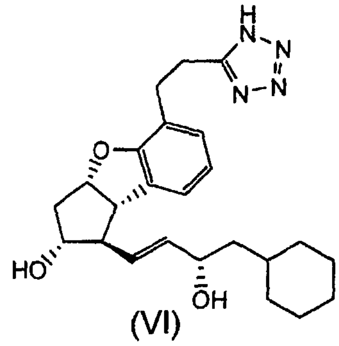

(VI)

**8.** The agent for modulating growth or generation of hair according to any one of claims 1 to 7, which is a promoting agent for growth or generation of hair.

**9.** Use of a prostaglandin EP4 receptor ligand for production of an agent for modulating growth or generation of hair.

**10.** The use according to claim 9, wherein the said prostaglandin EP4 receptor ligand is a 5,6,7-trinor-4,8-inter-m-phenylene $PGI_2$ derivative of said Formula (I) (wherein the definitions of the substituents in Formula (I) are the same as the definitions of the respective substituents in Formula (I) in claim 2) or a pharmacologically acceptable salt thereof.

**11.** The use according to claim 10, wherein the said 5,6,7-trinor-4,8-inter-m-phenylene $PGI_2$ derivative is represented by Formula (I) (wherein the definitions of the substituents in Formula (I) are the same as the definitions of the respective substituents in Formula (I) in claim 3) or a pharmacologically acceptable salt thereof.

**12.** The use according to claim 11, wherein the said 5,6,7-trinor-4,8-inter-m-phenylene $PGI_2$ derivative is represented by Formula (II) (wherein the definitions of the substituents in Formula (II) are the same as the definitions of the respective substituents in Formula (II) in claim 4) or a pharmacologically acceptable salt thereof.

**13.** The use according to claim 12, wherein the said 5,6,7-trinor-4,8-inter-m-phenylene $PGI_2$ derivative is represented by Formula (III) (wherein the definitions of the substituents in Formula (III) are the same as the definitions of the respective substituents in Formula (III) in claim 5) or a pharmacologically acceptable salt thereof.

**14.** The use according to claim 13, wherein the said 5,6,7-trinor-4,8-inter-m-phenylene $PGI_2$ derivative is represented by Formula (IV) (wherein the definitions of the substituents in Formula (IV) are the same as the definitions of the respective substituents in Formula (IV) in claim 6) or a pharmacologically acceptable salt thereof.

**15.** The use according to claim 14, wherein the said 5,6,7-trinor-4,8-inter-m-phenylene $PGI_2$ derivative is represented by the said Formula (V) or (VI).

**16.** The use according to any one of claims 9 to 15, wherein the said agent for modulating growth or generation of hair is an agent for promoting growth or generation of hair.

**17.** A method for modulating growth or generation of hair comprising administering a prostaglandin EP4 receptor ligand in an amount effective for modulating growth or generation of hair to human or an animal.

**18.** The method according to claim 17, wherein the said prostaglandin EP4 receptor ligand is a 5,6,7-trinor-4,8-inter-m-phenylene $PGI_2$ derivative of the said Formula (I) (wherein the definitions of the substituents in Formula (I) are the same as the definitions of the respective substituents in Formula (I) in claim 2) or a pharmacologically acceptable salt thereof.

**19.** The method according to claim 18, wherein the said 5,6,7-trinor-4,8-inter-m-phenylene $PGI_2$ derivative is represented by Formula (I) (wherein the definitions of the substituents in Formula (I) are the same as the definitions of the respective substituents in Formula (I) in claim 3) or a pharmacologically acceptable salt thereof.

**20.** The method according to claim 19, wherein the said 5,6,7-trinor-4,8-inter-m-phenylene $PGI_2$ derivative is represented by Formula (II) (wherein the definitions of the substituents in Formula (II) are the same as the definitions of the respective substituents in Formula (II) in claim 4) or a pharmacologically acceptable salt thereof.

**21.** The method according to claim 20, wherein the said 5,6,7-trinor-4,8-inter-m-phenylene $PGI_2$ derivative is represented by Formula (III) (wherein the definitions of the substituents in Formula (III) are the same as the definitions of the respective substituents in Formula (III) in claim 5) or a pharmacologically acceptable salt thereof.

**22.** The method according to claim 21, wherein the said 5,6,7-trinor-4,8-inter-m-phenylene $PGI_2$ derivative is represented by Formula (IV) (wherein the definitions of the substituents in Formula (IV) are the same as the definitions of the respective substituents in Formula (IV) in claim 6) or a pharmacologically acceptable salt thereof.

**23.** The method according to claim 22, wherein the said 5,6,7-trinor-4,8-inter-m-phenylene $PGI_2$ derivative is represented by the said Formula (V) or (VI).

**24.** The method according to any one of claims 17 to 23, wherein the said agent for modulating growth or generation of hair is an agent for promoting growth or generation of hair.

## INTERNATIONAL SEARCH REPORT

International application No.
PCT/JP01/02756

A. CLASSIFICATION OF SUBJECT MATTER
Int.Cl$^7$ A61K 7/06, A61P 17/14

According to International Patent Classification (IPC) or to both national classification and IPC

B. FIELDS SEARCHED

Minimum documentation searched (classification system followed by classification symbols)
Int.Cl$^7$ A61K 7/06-7/155, 31/343

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | EP, 572014, A1 (Toray Industries, Inc.), 01 December, 1993 (01.12.93), & JP, 5-331025, A & US 5508303 A | 2,10,16<br>1,3-9,11-15 |
| Y | Kiriyama, M. et al., "Ligand binding specificities of the eight types and subtypes of the mouse prostanoid receptors expressed in Chinese hamster ovary cells", Br. J. Pharmacol. (1997), Vol.122, No.2, pages 217 to 224, ISSN 0007-1188 | 1-16 |
| PA | WO, 00/54808, A (Toray Industries, Inc.), 21 September, 2000 (21.09.00), & EP, 1080728, A1 | 1-16 |
| A | JP, 10-287532, A (R Tec Ueno K.K.), 27 October, 1998 (27.10.98) (Family: none) | 1-16 |
| A | JP 61-218510 A (Daiichi Pharmaceutical Co., Ltd.), 29 September, 1986 (29.09.86) (Family: none) | 1-16 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 June, 2001 (12.06.01) | 26 June, 2001 (26.06.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP01/02756

C (Continuation). DOCUMENTS CONSIDERED TO BE RELEVANT

| Category* | Citation of document, with indication, where appropriate, of the relevant passages | Relevant to claim No. |
|---|---|---|
| A | WO, 96/03992, A1 (Loyola University of Chicago), 15 February, 1996 (15.02.96), & JP, 10-503522, A & US, 5578643, A & US, 5578640, A & US, 5605931, A | 1-16 |

Form PCT/ISA/210 (continuation of second sheet) (July 1992)

## INTERNATIONAL SEARCH REPORT

International application No.
PCT/JP01/02756

**Box I Observations where certain claims were found unsearchable (Continuation of item 1 of first sheet)**

This international search report has not been established in respect of certain claims under Article 17(2)(a) for the following reasons:

1. ☒ Claims Nos.: 17-24
because they relate to subject matter not required to be searched by this Authority, namely:

Claims 17 to 24 involve methods for treatment of the human body by therapy and thus involve a subject matter which this International Searching Authority is not required, under the provisions of Article 17(2)(a)(i) of the PCT and Rule 39(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)